(19) Europäisches Patentamt
European Patent Office
Office européen des brevets

(11) **EP 3 820 830 B1**

(12) **EUROPEAN PATENT SPECIFICATION**

(45) Date of publication and mention
of the grant of the patent:
**03.09.2025 Bulletin 2025/36**

(21) Application number: **19837528.9**

(22) Date of filing: **15.07.2019**

(51) International Patent Classification (IPC):
*C05D 9/00* (2006.01)   *C05D 9/02* (2006.01)
*C05G 3/00* (2020.01)   *A01N 59/02* (2006.01)
*C05F 11/10* (2006.01)   *A01N 25/14* (2006.01)
*C05G 5/10* (2020.01)

(52) Cooperative Patent Classification (CPC):
(C-Sets available)
**C05D 9/02; A01N 25/14; C05F 11/10**    (Cont.)

(86) International application number:
**PCT/IB2019/056001**

(87) International publication number:
**WO 2020/016730 (23.01.2020 Gazette 2020/04)**

(54) **AGRICULTURAL COMPOSITION CONTAINING SULPHUR AND METHOD OF OBTENTION THEREOF**

SCHWEFEL ENTHALTENDE LANDWIRTSCHAFTLICHE ZUSAMMENSETZUNG UND VERFAHREN ZU IHRER HERSTELLUNG

COMPOSITION AGRICOLE CONTENANT DU SOUFRE ET PROCÉDÉ POUR SA PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**KH TN**

(30) Priority: **14.07.2018 PCT/IB2018/055225**
**23.01.2019 IN 201921002743**

(43) Date of publication of application:
**19.05.2021 Bulletin 2021/20**

(73) Proprietor: **SML Limited**
**Mumbai 400069 (IN)**

(72) Inventors:
• **Sawant, Arun Vitthal**
**Thane, Maharashtra 421306 (IN)**
• **Puthenveetil Kunjukrishna Menon, Ramdas**
**Navi Mumbai, Maharashtra 400705 (IN)**

(74) Representative: **Appleyard Lees IP LLP**
**15 Clare Road**
**Halifax HX1 2HY (GB)**

(56) References cited:
EP-A2- 2 359 693      EP-A2- 2 359 693
WO-A1-03/053883      WO-A1-2012/131702
CA-A1- 2 663 119      GB-A- 2 513 232
US-A1- 2017 283 334

(52) Cooperative Patent Classification (CPC): (Cont.)

C-Sets
**C05D 9/02, C05F 11/10, C05G 3/40, C05G 3/50,
C05G 5/10;
C05D 9/02, C05F 11/10, C05G 5/10**

**Description**

## 1. FIELD OF THE INVENTION

The present invention relates to a novel agricultural composition for soil application to improve the plant growth, strength, health and nutritive value of crops. The invention is set out in the appended set of claims. The invention relates to a water dispersible granular composition for soil application comprising elemental sulphur in combination with at least one amino acid, their polymers, their salts, derivatives or mixtures thereof, at least one surfactant and at least one agrochemically acceptable excipient. The invention further relates to an agricultural composition in the form of a liquid suspension for soil application which comprises elemental sulphur in combination with at least one amino acid, their salts, their polymers or derivatives or mixtures thereof, at least one structuring agent and at least one agrochemically acceptable excipient. The water dispersible granular composition and liquid suspension composition can further include at least one micronutrient, their salts, derivatives or mixtures thereof. The invention relates to a process of preparing the agricultural composition and to a method of treating plants, seeds, crops, plant propagation material, locus, parts thereof or soil with the agricultural composition.

## 2. BACKGROUND

Nutrition and plant defence is the key element in the growth and development of crops. Poor availability of fertilizers or nutrients to plants results in lack of proper growth and the plants become more susceptible to attack by pests. Other problems associated with agriculture are the soil condition such as drought, biotic and abiotic stress, which tend to create low and uncertain yields. A large problem with several crop nutrients or fertilizers or plant growth promoting products is that, when applied it either remains in unavailable form and not adequately absorbed by the plant or it may rapidly leach through soil, due to either their rapid mobility in the soil or their physical form and characteristics. Thus, lesser amount of nutrition becomes available to plant and hence these products will have less nutrient use efficacy. In addition, leaching of nutrients may also contribute to groundwater contamination in regions with intensive agriculture. Thus, providing adequate nutrition in a manner such that there is a maximum uptake of nutrient by the plant along with protection to the crops pertaining to the environmental condition remains a great challenge. The use of fertilizer and micronutrient composition needs to be optimized and their uptake by crops on application needs to be improved in order to provide an economical result to the farmer and also reduce the burden on the environment.

The role of sulphur as an essential and a growth promoting nutrient and as fertilizer in agriculture has been long known. Sulphur is of great significance for synthesis of proteins and functioning of enzymes and it plays an important role in the defence of plants against stresses and pests. Sulphur is also necessary for the formation of chlorophyll, thus all plants need a continuous supply of sulphur from emergence to crop maturity for proper development of plants. However, sulphur is not readily available to plant and to make it available and translocated within plants, it requires oxidation by the soil microbes. Sulphur availability to plants is reduced if there is inadequate microbe population in the soil which leads to sulphur deficiency at any growth stage of crops and can eventually result in reduced crop growth and yield.

It is also known that a shortage of sulphur in the soil lowers utilization of the available soil nitrogen, thereby increasing nitrate leaching (Likkineni and Abrol, 1994). Sulphur interactions with nitrogen nutrients are directly related to the alteration of physiological and biochemical responses of crops. It is widely accepted that crops grown on soils deficient in Nitrogen exhibit very distinctive Nitrogen-deficiency symptoms such as poor growth, chlorosis, necrosis and causes disorder in many physiological/biochemical characteristics of plants.

Amino acids are known to play key physiological role in plants and acts as an important source of nitrogen. Plants can uptake amino acids more rapidly than inorganic nitrogen. Furthermore, amino acids are very useful to improve the production and the quality of the crop as they are building blocks of proteins, which exert manifold functions in plant metabolism, and as metabolites and precursors wherein they are involved with plant defence, stress management, biosynthesis of vitamins, enzymes, nucleotides and hormones, and as precursors of a huge variety of secondary compounds. However, it is important to provide these nutrients to the plant at the right stage for uptake and more so to make the nutrients available to the crops or the plant throughout the entire crop life cycle while also preventing or reducing leaching of the nutrients after application.

The prevalent practice is that sulphur and amino acids are applied separately and at different plant locus whereby sulphur is applied in soil whereas amino acids are applied by foliar application. However, this separate application of sulphur and amino acid at different time and at different plant locus is not only an uneconomical and time consuming approach to farmers but also fails to meet an effective nutrient requirement of the crops.

It was noted by the present inventors that absorption of sulphur and other nutrients by plant occurs best when applied along with amino acids. It was noticed by the present inventors that the presence of amino acid increases the microbial population in soil which assists in conversion of sulphur to sulphate thus making sulphur readily available to the plant which was hitherto not available. It was further noticed that application of sulphur and amino acid in combination results in reduction in soil pH thus creating an environment for better uptake of amino acids and other macro and micro nutrients by plant roots. It was noted that this synergy in the composition, resulted in both sulphur and amino acids getting rapidly absorbed through the roots by positive interactions between amino acids and nutrients in the rhizosphere.

Accordingly, the present inventors surprisingly found that the composition comprising elemental sulphur and amino acid is synergistic in nature and desirable for growth and development of crops. Such combination thus provides a nutrient use efficient composition comprising elemental sulphur, amino acid meeting the need of crops by providing multi nutritive solution with improved uptake by crops in a single application.

While there is no known formulation which comprises a mixture of elemental sulphur and amino acid, US20170283334 discloses mixtures containing micronutrient, polyelectrolyte and amino acid. US'334 discloses that the presence of such polyelectrolyte polymers like sodium alginate in the composition results in absorption of excess of water thus forming a thick paste. US'334 do not disclose water dispersible granule or liquid suspension composition of elemental sulphur and amino acid.

WO 03/053883 discloses a method for improving plant growth by application of a mixture M comprising a component a) of a1) 20 to 96% by weight of sulfur, a2) 4 to 80% by weight of a complexing agent and, if appropriate, one or more crop protectants b) and/or additives c).

EP2359693 discloses a fungicidal composition comprising of sulphur, potassium bicarbonate and mixture of amino acids and or peptides. They claim to provide a strong, safer and a better organic fungicidal solution replacing or reducing the dose of copper by adding potassium carbonate in the composition. EP '693 discloses a fungicidal composition in the form of wettable powder or fluid paste and does not disclose water dispersible granule or liquid suspension composition of elemental sulphur and amino acid.

It may be noted that compositions in the form of wettable powder or a fluid paste not only have issues with respect to practical application like being unpourable and generation of dust but also pose risk to the users mostly because of eye irritation, inhalation risk and skin irritation. Such formulations are also not easily dispersible and tend to clog the nozzles when applied via drip, making it unsuitable for use in irrigation system. Further, such compositions have also been found to have poor suspensibility which leads to random distribution of active ingredient on the target area. Such randomly distributed active would cause undesirable effect and pose a problem in effective delivery of nutrients to the plant or crop and are also required to be used in large amounts.

There exist no disclosures in the art directing towards a specific composition comprising elemental sulphur in combination with amino acid in the form of a water dispersible granule or liquid suspension which can be effectively used to meet the requirement of plants or enhance nutrient use efficiency or nutrient uptake and address the drawbacks discussed above.

Moreover, preparing an effective and stable water dispersible granule on account of the highly explosive nature of sulphur composition comprising of micronized sulphur and amino acid was found to be a major challenge. Amino acids being hydrophilic in nature required higher temperature conditions for preparing a dry composition. Since formulating a micronized composition containing a high concentration of elemental sulphur and amino acids required drying at higher temperature conditions, it carried with it an enhanced risk of sulphur explosion and fire hazards.

Inventors of the present invention were successful in overcoming the associated risk and were able to formulate a water dispersible granular and stable liquid suspension composition of elemental sulphur and amino acids overcoming these drawbacks and preparing a concentrated yet dispersible composition with reduced particle size.

In addition, it was surprisingly noted that the composition of the present invention when formulated at a specific particle size of 0.1-20 microns, made sulphur, nutrients and amino acids readily available for uptake by the plants and increase the overall yield. The composition of the present invention also exhibits superior physical characteristics such as suspensibility, dispersibility, flowability, wettability, stability and improved viscosity resulting in better pourability. The compositions of the present invention also demonstrated superior performance under accelerated storage conditions and also effective usage in drip irrigation.

To summarize, application of elemental sulphur and amino acid, their polymers, salts, or derivatives or mixtures thereof and further micronutrients in the form of water dispersible granules and liquid suspension help alter the soil conditions and subsequently improve the uptake of nutrients in the crop and demonstrate excellent field efficacy at reduced dosage. Furthermore, the application of sulphur and amino acids in the form of the present composition prevents leaching of nutrients, reduces dosage, improves yield, protein synthesis, quality, vitality and vigour of crops, boost energy metabolism, stimulate phyto-hormones, improves nutrient content of the crops, helps in managing disease resistance and stress management providing a stronger and nutritionally rich crop.

## 3. SUMMARY OF THE INVENTION

The inventors have determined that a water dispersible granular agricultural composition for soil application comprising effective amounts of elemental sulphur, at least one amino acid, their polymers or salts or derivatives or mixtures thereof, at least one surfactant, and at least one agrochemically acceptable excipient provides higher yield in various crops and improves plant physiological parameters, defence mechanism of the plant and also finds a direct use in micro irrigation systems. The water dispersible granules of the invention is as defined in claim 1. The water dispersible granules comprise one or more amino acid, their polymers, salts or derivatives or mixtures thereof in a concentration range of 0.1% to 70% by weight of the total composition, elemental sulphur in a concentration range of 20% to 99% by weight of the total composition, at least one surfactant in the range of 0.1%-60%by weight of the total composition and at least one agrochemical excipient. Further, the water dispersible granular agricultural composition comprises granules in the size range of 0.1-5 mm which disperses into particles in the size range of 0.1 micron to 20 microns.

Furthermore, the inventors of the application have also found that an agricultural composition in the form of a liquid suspension for soil application is as defined in claim 2, comprising effective amounts of elemental sulphur, at least one amino acid, their polymers, salts or derivatives or mixtures thereof; at least one structuring agent and at least one agrochemically acceptable excipient demonstrated high yield in certain crops and also finds a direct use in micro irrigation systems. The liquid suspension comprises amino acid, their polymers, salts or derivatives or mixtures thereof in a concentration range of 0.1% to 70% by weight of the total composition, elemental sulphur in the range of 1% to 65% by weight of the total composition, at least one structuring agent in the range of 0.01% to 5%by weight of total composition and at least one agrochemical excipient; the composition comprises particles in the size range of 0.1-20microns.

The agricultural composition in the form of a water dispersible granules and liquid suspension for soil application further includes at least one micronutrient in the concentration of 0.1% to 70% by weight of the total composition.

Furthermore, the invention relates to a process of preparing the agricultural composition comprising effective amount of at least one amino acid, their polymers, salts or derivatives or mixtures thereof with elemental sulphur and at least one agrochemically acceptable excipient; the composition having a particle size in the range of from 0.1-20microns.

The invention also relates to a method of treating the plants, seeds, crops, plant propagation material, locus, parts thereof or the soil with the agricultural composition comprising an effective amount of elemental sulphur and at least one amino acid, their polymers, salts or derivatives or mixtures thereof; and at least one agrochemically acceptable excipient.

Quite advantageously, the compositions are applied to the soil, through broadcasting or bend/side placement, drenching, drilling or through micro irrigation such as drip or trickle irrigation. The latter case of drip or trickle irrigation further optimizes farming practices, which are greatly challenged by an ever-increasing labour and water shortage. Thus, the compositions of the invention are used in all possible ways of application, as per the convenience of the user.

According to an embodiment, the invention further relates to a method of improving plant health or yield by treating at least one of a plant, a plant propagation material, locus or parts therof, a seed, seedling or surrounding soil with the composition of claims 1 or 2.

Mention has also been done to improving the crop health, improving the crop nutrition, fortifying or strengthening the crop, protecting the crop, enhancing the crop yield or conditioning the soil, treating at least one of seeds, seedling, crops, a plant, plant propagation material, locus, parts thereof or to the surrounding soil with an agricultural composition comprising effective amounts of elemental sulphur and at least one amino acid, their salts, polymers, derivatives or mixtures thereof, at least one micronutrient and at least one agrochemically acceptable excipient.

It was observed that the composition is synergistic in nature, improve the uptake of sulphur and other nutrient by plant, improves soil microbial properties and exhibits good physical and chemical properties. It was further noted that the composition is readily dispersible, good suspension, non- viscous, readily pourable, and stable even at extended storage and under higher temperatures.

4. DESCRIPTION OF THE DRAWINGS:

For a more complete understanding of the invention, reference should now be made to the embodiments illustrated in greater detail in the accompanying drawings and described by way of embodiments of the invention.

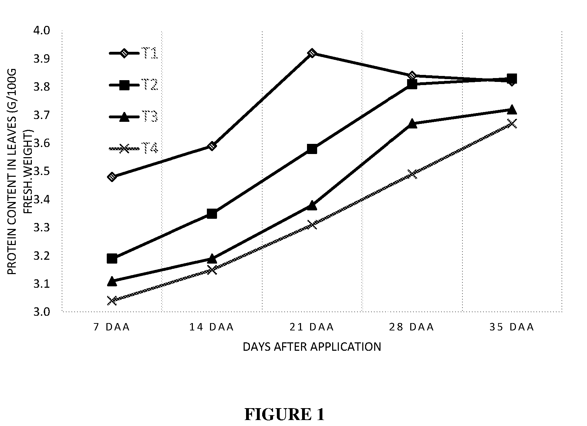

FIGURE 1: Graphical Representation to study the effect of water dispersible granular composition of Elemental sulphur and Amino acids with micronutrients at different particle size on protein uptake by plant leaves.

5. DETAILED DESCRIPTION OF THE INVENTION

In describing the embodiment of the invention, specific terminology is chosen for the sake of clarity. However, it is not intended that the invention be limited to the specific terms so selected and it is to be understood that such specific terms include all technical equivalents that operate in a similar manner to accomplish a similar purpose. It is understood that any numerical range recited herein is intended to include all subranges subsumed. Also, unless denoted otherwise percentage of components in a composition are presented as weight percent.

A water dispersible granule is defined as a formulation consisting of granules to be applied after disintegration and dispersion in water. As described herein, "WG" or "WDG" refer to water dispersible granules.

According to the invention, the term liquid suspension encompasses "aqueous suspension" or aqueous dispersion" or "suspension concentrates" or "suspo emulsion" or an SC composition. Liquid suspension can be defined as composition wherein solid particles are dispersed or suspended in a liquid. The liquid as a vehicle can be water and/or a water miscible solvent.

Nutrient use efficiency (NUE) is defined as a measure of how well plants use the available mineral nutrients. Improvement of NUE is an essential pre-requisite for expansion of crop production into marginal lands with low nutrient availability but also a way to reduce use of inorganic fertilizer.

The invention relates to an agricultural composition for soil application in the form of water dispersible granules which comprises 0.1%-70% by weight of at least one amino acid, their polymers, salts or derivatives or mixtures thereof and 20% to 99% by weight of elemental Sulphur; at least one agrochemically acceptable excipient. The invention also relates to an agricultural composition in the form of liquid suspension for soil application which includes 0.1% to 70% by weight of at least one amino acid, their polymers, salts or derivatives or mixtures thereof and 1% to 65% by weight of elemental Sulphur; at least one agrochemically acceptable excipient. The agricultural composition comprise of particles in the size range of from 0.1 of 0.1 to 20 microns and exhibits improved dispersibility and suspensibility.

According to an embodiment, the agricultural composition is in a solid form or a liquid form. For e.g, the agricultural composition may be in the form of aqueous suspension, suspo-emulsion, suspension concentrate, aqueous dispersion, water dispersible granules, seed dressings or emulsions for seed treatment, and combinations thereof.

The agricultural composition comprises elemental sulphur.

The water dispersible granules comprise elemental sulphur in the range of 20% to 99% by weight of the total composition. According to an embodiment, the water dispersible granules comprise elemental sulphur in the range of 40% to 99% by weight of the total composition. According to an embodiment, the water dispersible granules comprise elemental sulphur in the range of 20% to 80% by weight of the total composition. According to an embodiment, the water dispersible granules comprise elemental sulphur in the range of 20% to 50% by weight of the total composition.

The liquid suspension composition comprises elemental sulphur in the range of 1% to 65% by weight of the total composition. According to an embodiment, the liquid suspension composition comprises elemental sulphur in the range of 1% to 50% by weight of the total composition. According to an embodiment, the liquid suspension composition comprises elemental sulphur in the range of 1% to 40% by weight of the total composition.

The elemental sulphur and amino acid in the composition not only acts as a fertilising agent but also helps to reduce the soil pH and foster better uptake of amino acids and other nutrients. It was surprisingly observed that the effect of the combination of elemental sulphur and amino acid, their polymers, salts or derivatives or mixtures thereof in the form of water dispersible granule and as well as liquid suspension at a particle size range of 0. 1-20microns is synergistic and showed superior field efficacy in terms of growth and development of crops. This superior efficacy was not observed with individual application of sulphur and amino acids.

The composition comprises at least one or more of amino acids. The amino acids can include one or more of essential, non-essential amino acids, non-proteogenic amino acids, oligomers, homopolymers, random co-polymers of amino acids, peptides, plant and animal proteins, hydrolysed proteins, polyamino acids, salts or derivatives thereof or mixtures thereof. The amino acids comprises one or more of but are not limited to alanine, arginine, aspartic acid, asparagine, citrulline, leucine, lysine, isoleucine, cysteine, glutamic acid, glutamine, glycine, gaba-aminobutyric acid, histidine, methionine, ornithine, proline, phenylalanine, serine, selenocysteine, valine, taurine, tyrosine, theanine, threonine, tryptophan, tyramine or salts or derivatives thereof. The hydrolysed proteins comprises one or more of but not limited to soya protein, whey protein, fig protein, fish protein, pea protein, maize protein, casein protein, sunflower protein, barley protein, egg protein, rapeseed protein, rice protein, gelatin, chicken protein or mixtures thereof. The protein hydrolysates can be from vegetable, animal or microbial source. The polymers of amino acids include one or more of but not limited to polylysine, polyornithine, polyarginine, polylactic acid, polyglutamic acid, polylactide, polythreonine, polyproline, polyhistidine, polytyrosine. However those skilled in the art will appreciate that it is possible to utilize other amino acids without departing from the scope of present invention. The amino acids, their polymers, salts or derivatives or peptides or protein hydrolysates are commercially manufactured and sourced through various companies.

According to a further embodiment, the composition preferably comprises amino acids selected from glycine, proline, arginine, lysine, glutamine, histidine, tryptophan, glutamic acid, methionine, cysteine, polyglutamic acid, plant and animal protein, soya protein hydrolysate, whey protein hydrolysate, fish protein hydrolysate, their polymers, salts or derivatives or mixtures thereof.

The amino acid, their polymers, salts or derivatives or mixtures thereof are present in a concentration range of from 0.1% to 70% by weight of the total composition. According to an embodiment, the amino acid, their polymers, salts or derivatives or mixtures thereof are present in a concentration range of from 0.1% to 60% by weight of the total composition. According to an embodiment, the amino acid, their polymers, salts or derivatives or mixtures thereof are present in a concentration range of from 0.1% to 50% by weight of the total composition. According to an embodiment, the amino acid, their polymers, salts or derivatives or mixtures thereof are present in a concentration range of from 0.1% to 40% by weight of the total composition. According to an embodiment, the amino acid, their polymers, salts or derivatives or mixtures thereof are present in a concentration range of from 0.1% to 20% by weight of the total composition. According to an embodiment, the amino acid, their polymers, salts or derivatives or mixtures thereof are present in a concentration range of from 0.1% to 10% by weight of the total composition. According to an embodiment, the amino acid, their polymers, salts or derivatives or mixtures thereof are present in a concentration range of from 0.1% to 5% by weight of the total composition.

According to an embodiment, the micronutrients comprise water soluble or water insoluble micronutrients. According to an embodiment, the micronutrients comprise one or more of zinc, boron, calcium, iron, magnesium, copper, manganese, silicon, cobalt, molybdenum, chromium, vanadium, selenium, nickel, iodine, in their elemental form, or salts, complexes, derivatives or mixtures thereof. According to a further embodiment, the micronutrients preferably includes one or more of zinc, iron, copper, molybdenum, manganese, magnesium in their elemental form, or salts, complexes, derivatives or mixtures thereof. However, the above list of micronutrients is exemplary and not meant to limit the scope of the invention.

According to an embodiment, the micronutrients are present in the range of from 0.1% to 70% by weight of the total composition. According to an embodiment, the micronutrients are present in the range of from 0.1% to 40% by weight of the total composition. According to an embodiment, the micronutrients are present in the range of from 0.1% to 20% by weight of the total composition.

According to an embodiment, the molar ratio of amino acids and the metal ions of water soluble micronutrient is 2:1

According to an embodiment, the weight ratio of amino acids and the metal ions of water soluble micronutrient can be 2:1 to 38:1 by weight of the total composition. According to an embodiment, the weight ratio of amino acids and the metal ions of water soluble micronutrient can be 2:1 by weight of the total composition. According to an embodiment, the weight ratio of amino acids and the metal ions of water soluble micronutrient can be 3:1 by weight of the total composition. According to an embodiment, the weight ratio of amino acids and the metal ions of water soluble micronutrient can be 4:1 by weight of the total composition. According to an embodiment, the weight ratio of amino acids and the metal ions of water soluble micronutrient can be 5:1 by weight of the total composition. According to an embodiment, the weight ratio of amino acids and the metal ions of water soluble micronutrient can be 6:1 by weight of the total composition. According to an embodiment, the weight ratio of amino acids and the metal ions of water soluble micronutrient can be 7:1 by weight of the total composition. According to an embodiment, the weight ratio of amino acids and the metal ions of water soluble micronutrient can be 8:1 by weight of the total composition. According to an embodiment, the weight ratio of amino acids to the metal ions of water soluble micronutrient can be 10:1 by weight of the total composition. According to an embodiment, the weight ratio of amino acids to the metal ions of water soluble micronutrient can be present 12:1 by weight of the total composition. According to an embodiment, the weight ratio of amino acids to the metal ions of water soluble micronutrient can be 14:1 by weight of the total composition. According to an embodiment, the weight ratio of amino acids to the metal ions of water soluble micronutrient can be 15:1 by weight of the total composition. According to an embodiment, the weight ratio of amino acids to the metal ions of water soluble micronutrient can be in a ratio 16:1 by weight of the total composition. According to an embodiment, the weight ratio of amino acids to the metal ions of water soluble micronutrient can be 17:1 by weight of the total composition. According to an embodiment, the weight ratio of amino acids to the metal ions of water soluble micronutrient can be 19:1 by weight of the total composition. According to an embodiment, the weight ratio of amino acids to the metal ions of water soluble micronutrient can be 20:1 by weight of the total composition. According to an embodiment, the weight ratio of amino acids to the metal ions of water soluble micronutrient can be 21:1 by weight of the total composition. According to an embodiment, the weight ratio of amino acids to the metal ions of water soluble micronutrient can be 22:1 by weight of the total composition. According to an embodiment, the weight ratio of amino acids to the metal ions of water soluble micronutrient can be 23:1 by weight of the total composition. According to an embodiment, the weight ratio of amino acids to the metal ions of water soluble micronutrient can be 25:1 by weight of the total composition. According to an embodiment, the weight ratio of amino acids to the metal ions of water soluble micronutrient can be 28:1 by weight of the total composition. According to an embodiment, the weight ratio of amino acids to the metal ions of water soluble micronutrient can be 30:1 by weight of the total composition. According to an embodiment, the weight ratio of amino acids to the metal ions of water soluble micronutrient can be 32:1 by weight of the total composition. According to an embodiment, the weight ratio of amino acids to the metal ions of water soluble micronutrient can be 34:1 by weight of the total composition. According to an embodiment, the weight ratio of amino acids to the metal ions of water soluble micronutrient can be 35:1 by weight of the total composition. According to an embodiment, the weight ratio of amino acids to the metal ions of water soluble micronutrient can be 37:1.

The composition may further comprise plant growth promoters namely one or more of vitamins, bio-stimulants, organic acids or salts, plant growth regulator, complexes or derivatives or mixtures thereof. The plant growth promoters includes humic acid, ascorbic acids, fulvic acid, lactic acid, oxalic acid, phytic acid, fumaric acid, gibberelic acid, auxins, citric acid, N-acetyl thiazolidine-4 carboxylic acid, paclobutrazol, triacontanol, or mixtures thereof. According to an embodiment, the plant growth promoter is present in the concentration of 0.1-60%, preferably 0.1-20% w/w of the total composition. However, the above list of plant growth promoter is exemplary and not meant to limit the scope of the invention. The plant growth promoters are commercially manufactured and sourced through various vendors who manufacture and sell these products on commercial scale..

The agricultural composition is in the form of water dispersible granules.

The agricultural composition in the form of water dispersible granules comprises at least one of amino acid, their polymers, salts or derivatives or mixtures thereof in the range of 0.1% to 70% by weight of the total composition and elemental sulphur in the range of 20% to 99% by weight of the total composition, one or more surfactant in the range of 0.1% to 60% by weight of the total composition and at least one agrochemically acceptable excipient. The agricultural composition optionally can comprise at least one micronutrient.

According to an embodiment, the agricultural composition in the form of water dispersible granules comprises at least one amino acids, their polymers, salts or derivatives or mixtures thereof in the range of 0.1% to 70% by weight of the total composition and elemental sulphur in the range of 20% to 99% by weight of the total composition along with at least one micronutrient in the concentration of 0.1 to 70% by weight of the total composition. The composition also comprises one or more surfactant in the range of 0.1% to 60% by weight of the total composition and at least one agrochemical excipient.

According to an embodiment, the agricultural composition in the form of water dispersible granules comprises elemental sulphur in the range of from 20% to 99% by weight of the total composition in combination with at least one amino acid, their polymers, salts or derivatives or mixtures thereof selected from alanine, arginine, histidine, glutamine, glycine, lysine, tryptophan, proline in the range of 0.1% to 70% by weight of the total composition and at least one micronutrient selected from zinc, iron, manganese, magnesium, copper, boron, molybdenum their salts or derivatives or mixtures thereof present in the concentration of 0.1% to 70% by weight of the total composition with one or more surfactants in the range of 0.1% to 30% by weight of the total composition and at least one agrochemical excipient.

According to an embodiment, the agricultural composition in the form of water dispersible granules comprises elemental sulphur in the range of from 20% to 99% by weight of the total composition in combination with at least one protein hydrolysate in the range of 0.1% to 70% by weight of the total composition and at least one micronutrient selected from zinc, iron, manganese, magnesium, copper, boron, molybdenum their salts or derivatives or mixtures thereof present in the concentration of 0.1% to 70% by weight of the total composition with one or more surfactants in the range of 0.1% to 30% by weight of the total composition and at least one agrochemical excipient.

According to an embodiment, the agricultural composition in the form of water dispersible granules comprises elemental sulphur in the range of from 20% to 99% by weight of the total composition in combination with at least one protein hydrolysate in the range of 0.1% to 70% by weight of the total composition and at least one plant growth promoter selected from humic acid or fulvic acid or triacontanol present in the concentration of 0.1% to 20% by weight of the total composition with one or more surfactants in the range of 0.1% to 30% by weight of the total composition and at least one agrochemical excipient. The agricultural composition optionally can comprise at least one micronutrient.

According to an embodiment, the weight ratio of elemental sulphur to one or more of amino acid, their polymers, salts or derivatives or mixtures in a water dispersible granular form is 990:1 to 1:3.5. According to an embodiment, the weight ratio of elemental sulphur to one or more of amino acid, their polymers, salts or derivatives or mixtures in a water dispersible granular form is 500:1. According to an embodiment, the weight ratio of elemental sulphur to one or more of amino acid, their polymers, salts or derivatives or mixtures in a water dispersible granular form is 100:1. According to an embodiment, the weight ratio of elemental sulphur to one or more of amino acid, their polymers, salts or derivatives or mixtures in a water dispersible granular form is 50:1. According to an embodiment, the weight ratio of elemental sulphur to one or more of amino acid, their polymers, salts or derivatives or mixtures in a water dispersible granular form is 10:1. According to an embodiment, the weight ratio of elemental sulphur to one or more of amino acid, their polymers, salts or derivatives or mixtures in a water dispersible granular form is 9:1.According to an embodiment, the weight ratio of elemental sulphur to one or more of amino acid, their polymers, salts or derivatives or mixtures in a water dispersible granular form is 8:1. According to an embodiment, the weight ratio of elemental sulphur to one or more of amino acid, their polymers, salts or derivatives or mixtures in a water dispersible granular form is 7:1. According to an embodiment, the weight ratio of elemental sulphur to one or more of amino acid, their polymers, salts or derivatives or mixtures in a water dispersible granular form is 6:1. According to an embodiment, the weight ratio of elemental sulphur to one or more of amino acid, their polymers, salts or derivatives or mixtures in a water dispersible granular form is 5:1. According to an embodiment, the weight ratio of elemental sulphur to one or more of amino acid, their polymers, salts or derivatives or mixtures in a water dispersible granular form is 4:1. According to an embodiment, the weight ratio of elemental sulphur to one or more of amino acid, their polymers, salts or derivatives or mixtures in a water dispersible granular form is 3:1. According to an embodiment, the weight ratio of elemental sulphur to one or more of amino acids, their polymers, salts or derivatives or mixtures in a water dispersible granular form is 2.5:1. According to an embodiment, the weight ratio of elemental sulphur to one or more of amino acid, their polymers, salts or derivatives or mixtures in a water dispersible granular form is 2.2:1. According to an embodiment, the weight ratio of elemental sulphur to one or more of amino acid, their polymers, salts or derivatives or mixtures in a water dispersible granular form is 2:1. According to an embodiment, the weight ratio of elemental sulphur to one or more of amino acid, their polymers, salts or derivatives or mixtures in a water dispersible granular form is 1.5:1. According to an embodiment, the weight ratio of elemental sulphur to one or more of amino acid, their polymers, salts or derivatives or mixtures in a water dispersible granular form is 1:1. According to an embodiment, the weight ratio of elemental sulphur to one or more of amino acid, their polymers, salts or derivatives or mixtures in a water dispersible granular form is 1:3. According to an embodiment, the weight ratio of elemental sulphur to one or more of amino acid, their polymers, salts or derivatives or mixtures in a water dispersible granular form is 1:2.

According to an embodiment, the agricultural composition is in the form of a liquid suspension.

The agricultural composition in the form of liquid suspension comprises at least one amino acid, their salts, derivatives or mixtures thereof in the range of 0.1% to 70% by weight of the total composition and elemental sulphur in the range of 1% to 65% by weight of the total composition and one or more structuring agents in the range of 0.01% to 5% by weight of the total composition and one or more agrochemically acceptable excipients. The agricultural composition optionally can comprise at least one micronutrient.

According to an embodiment, the agricultural composition in the form of liquid suspension comprises at least one of amino acid, their polymers, salts or derivatives or mixtures thereof in the range of 0.1% to 70% by weight of the total composition and elemental sulphur in the range of 1% to 65% by weight of the total composition along with at least one micronutrient in the range of 0.1% to 70% by weight of the total composition. The composition also includes one or more structuring agents in the range of 0.01% to 5% by weight of the total composition and one or more of agrochemically acceptable excipients.

According to an embodiment, the agricultural composition in the form of liquid suspension comprises elemental sulphur in the range of from 1% to 65% by weight of the total composition in combination with at least one amino acid, salts or derivatives or mixtures thereof selected from alanine, arginine, histidine, glutamine, glycine, lysine, tryptophan, proline in the range of 0.1% to 70% by weight of the total composition and at least one micronutrient selected from zinc, iron, manganese, magnesium, copper, boron, molybdenum their salts or derivatives or mixtures thereof present in the concentration of 0.1% to 70% by weight of the total composition and one or more structuring agent in the range of 0.01% to 5% by weight of the total composition.

According to an embodiment, the agricultural composition in the form of liquid suspension comprises elemental sulphur in the range of from 1% to 65% by weight of the total composition in combination with at least one protein hydrolysate in the range of 0.1% to 70% by weight of the total composition and at least one micronutrient selected from zinc, iron, manganese, magnesium, copper, boron, molybdenum their salts or derivatives or mixtures thereof present in the concentration of 0.1% to 70% by weight of the total composition with one or more of structuring agent in the range of 0.01% to 5% by weight of the total composition.

According to an embodiment, the agricultural composition in the form of liquid suspension comprises elemental sulphur in the range of from 1% to 65% by weight of the total composition in combination with at least one protein hydrolysate in the range of 0.1% to 70% by weight of the total composition and at least one plant growth promoter selected from humic acid, fulvic acid, triacontanol present in the concentration of 0.1% to 20% by weight of the total composition with one or more of structuring agent in the range of 0.01% to 5% by weight of the total composition. The agricultural composition optionally can comprise at least one micronutrient.

According to an embodiment, the weight ratio of elemental sulphur to one or more of amino acid, their polymers, salts or derivatives or mixtures thereof in a liquid suspension is 650:1 to 1:70. According to an embodiment, the weight ratio of elemental sulphur to one or more of amino acid, their polymers, salts or derivatives or mixtures thereof in a liquid suspension is 100:1. According to an embodiment, the weight ratio of elemental sulphur to one or more of amino acid, their polymers, salts or derivatives or mixtures in a liquid suspension is 50:1. According to an embodiment, the weight ratio of elemental sulphur to one or more of amino acid, their polymers, salts or derivatives or mixtures in a liquid suspension is 10:1. According to an embodiment, the weight ratio of elemental sulphur to one or more of amino acid, their polymers, salts or derivatives or mixtures in a liquid suspension is 9:1. According to an embodiment, the weight ratio of elemental sulphur to one or more of amino acid, their polymers, salts or derivatives or mixtures in a liquid suspension is 8:1. According to an embodiment, the weight ratio of elemental sulphur to one or more of amino acid, their polymers, salts or derivatives or mixtures in a liquid suspension is 7:1. According to an embodiment, the weight ratio of elemental sulphur to one or more of amino acid, their polymers, salts, derivatives or mixtures in a liquid suspension is 6:1. According to an embodiment, the weight ratio of elemental sulphur to one or more of amino acid, their polymers, salts or derivatives or mixtures in a liquid suspension is 5:1. According to an embodiment, the weight ratio of elemental sulphur to one or more of amino acid, their polymers, salts or derivatives or mixtures in a liquid suspension is 4:1. According to an embodiment, the weight ratio of elemental sulphur to one or more of amino acid, their polymers, salts or derivatives or mixtures in a liquid suspension is 3:1. According to an embodiment, the weight ratio of elemental sulphur to one or more of amino acid, their polymers, salts or derivatives or mixtures in a liquid suspension is 2.5:1. According to an embodiment, the weight ratio of elemental sulphur to one or more of amino acid, their polymers, salts or derivatives or mixtures in a liquid suspension is 2.2:1. According to an embodiment, the weight ratio of elemental sulphur to one or more of amino acid, their polymers, salts or derivatives or mixtures in a liquid suspension is 2:1. According to an embodiment, the weight ratio of elemental sulphur to one or more of amino acid, their polymers, salts or derivatives or mixtures in a liquid suspension is 1.5:1. According to an embodiment, the weight ratio of elemental sulphur to one or more of amino acid, their polymers, salts or derivatives or mixtures in a liquid suspension is 1:1. According to an embodiment, the weight ratio of elemental sulphur to one or more of amino acid, their polymers, salts or derivatives or mixtures in a liquid suspension is 1:14. According to an embodiment, the weight ratio of elemental sulphur to one or more of amino acid, their polymers, salts or derivatives or mixtures in a liquid suspension is 1:7.

According to an embodiment, the agricultural composition in the form of liquid suspension and water dispersible granules comprise particles in the size range of 0.1 micron to 20 microns, preferably, particles in the size range of 0.1 micron to 15 microns and most preferably in the range of 0.1 to 10 microns. Better uptake of amino acid and sulphur is made available to the crops at a particle size range of about 0.1 to 20 microns. Thus, the particle size range of 0.1 to 20 microns of the agricultural composition was found to be important not only in terms of ease of application but also in terms of efficacy.

According to an embodiment, the agricultural composition is in the form of water dispersible granules, wherein the granules are in the size range of 0.1 to 5 mm. According to an embodiment, the water dispersible granules are in the size range of 0.1 to 4 mm. According e o an embodiment, the water dispersible granules are in the size range of 0.1 to 3 mm. According to an embodiment, the water dispersible granules are in the size range of 0.1 to 2.5 mm. According to an embodiment, preferably the water dispersible granules are in the size range of 0.1 to 2 mm. Preferably, the water dispersible granules are in the size range of 0.1 to 1.5 mm. Preferably, the water dispersible granules are in the size range of 0.1 to 1 mm. Most preferably, the water dispersible granules are in the size range of 0.1 to 0.5 mm. According to an embodiment, the water dispersible granular agricultural composition, wherein the composition is in the form of microgranules. The granule comprises particles in the size range of 0.1 to 20 microns.

According to an embodiment, the agricultural composition may optionally comprise at least one fertilizer. Fertilizers are simply crop nutrients applied to agricultural fields to supplement required elements found naturally in the soil. The soil tends to lose its fertility due to continuous nutrient uptake by crops, run off losses with water, leaching, volatilization of nutrients and erosion of soil as a result of which the requirement of the crop is not met. Application of fertilizers not only assist in increasing yield and promoting healthy crop but also helps in development of defence against the pest and disease attack. Thus, application of optimum amount and type of fertilizer to the crops is crucial in meeting the nutrient requirement of the crop.

According to another embodiment, the fertilizers include single nutrient fertilizers, multi nutrient fertilizers, binary fertilizers, compound fertilizers, organic fertilizers or mixtures thereof. However, those skilled in the art will appreciate that it is possible to utilize other fertilizers known in the art, without departing from the scope of the invention.

According to yet another embodiment, the fertilizer comprises one or more of water soluble fertilizer or water insoluble fertilizer, or salts or complexes or derivatives, or mixtures thereof.

According to further embodiment, the fertilizers includes nitrogen, phosphate, potash, ammonia, ammonium nitrate, urea, sodium nitrate, potassium chloride, potassium sulfate, potassium carbonate, potassium nitrate, monoammonium phosphate, diammonium phosphate, calcium ammonium nitrate, super phosphates, phosphorgypsum, triple super phosphates, NPK fertilizers or salts or complexes or derivatives, or mixtures thereof. However, those skilled in the art will appreciate that it is possible to use other fertilizers without departing from the scope of the present invention. The fertilizers are commercially manufactured and available through various companies.

According to an embodiment, the fertilizer is present in the range of 1% to 90% by weight of the total composition. Preferably, the fertilizer is present in the range of 1% to 40% by weight of the total composition.

According to an embodiment, the agricultural composition further optionally comprises one or more of pesticidal actives. According to an embodiment, pesticidal active ingredient is present in an amount of 0.1% to 90% by weight of the composition. According to further embodiment, pesticidal active ingredient is present in an amount of 0.1% to 60% by weight of the composition. According to further embodiment, pesticidal active ingredient is present in an amount of 0.1% to 40% by weight of the composition.

According to an embodiment, the agricultural composition further comprises at least one agrochemically acceptable excipient. According to further embodiment, the agrochemically acceptable excipient comprises one or more of surfactants, dispersing agents, wetting agents, binders or binding agents, disintegrating agents, fillers or carriers or diluents, emulsifiers, hydrophobic agents, solvents, spreading agents, coating agents, buffers or pH adjusters or neutralizing agents, antifoaming agents or defoamers, penetrants, preservatives, ultraviolet absorbents, UV ray scattering agents, stabilizers, pigments, colorants, structuring agents, suspending agents or suspension aid agents, humectants, sticking agents, antifreezing agent or freeze point depressants, and mixtures thereof. However, those skilled in the art will appreciate that it is possible to utilize additional agrochemically acceptable excipients without departing from the scope of the present invention. The agrochemically acceptable excipients are commercially manufactured and sourced through various companies.

According to an embodiment, the agricultural composition in the form of water dispersible granules further comprises at least one agrochemical excipient. According to further embodiment, the agrochemically acceptable excipients which are used in water dispersible granular formulation include at least one disintegrating agents, wetting agents, binders or fillers or carriers or diluents, buffers or pH adjusters or neutralizing agents, antifoaming agents, anticaking agent, hydrophobic agents, spreading agents, penetrating agent, sticking agent. However, those skilled in the art will appreciate that it is possible to utilize additional agrochemically acceptable excipients without departing from the scope of the present invention.

According to an embodiment, the agricultural composition in the form of liquid suspension further comprises at least one agrochemical excipient. According to further embodiment, the agrochemically acceptable excipients which are used in suspension concentrates or liquid suspension or aqueous suspension formulations include at least one surfactant, dispersing agent, wetting agent, humectants, solvents, spreading agent, suspending agents or suspension aid, penetrating agent, sticking agents, ultraviolet absorbents, UV ray scattering agents, preservatives, stabilizer, buffers or pH adjusters or neutralizing agents, antifreezing agent or freeze point depressants, antifoaming agents, anticaking agent. However, those skilled in the art will appreciate that it is possible to utilize additional agrochemically acceptable excipients without departing from the scope of the present invention.

According to an embodiment, the agrochemical excipients are present in a concentration range of 1% to 90% by weight of the total composition. According to an embodiment, the agrochemical excipients are present in a concentration range of 1% to 70% by weight of the total composition. According to an embodiment, the agrochemical excipients are present in a concentration range of 1% to 50% by weight of the total composition. According to an embodiment, the agrochemical excipients are present in a concentration range of 1% to 30% by weight of the total composition.

According to an embodiment, the surfactants include one or more of emulsifiers, wetting agents and dispersing agents. According to an embodiment, the surfactants which are used in the agricultural composition include one or more of anionic, cationic, non-ionic, amphoteric and polymeric surfactants.

The anionic surfactants include one or more of, but not limited to a salt of fatty acid, a benzoate, a polycarboxylate, a salt of alkylsulfuric acid ester, alkyl ether sulfates, an alkyl sulfate, an alkylarylsulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyletherdisulfonate, a polystyrene sulfonate, a salt of alkylphosphoric acid ester, an alkylaryl phosphate, a styrylaryl phosphate, sulfonate docusates, a salt of polyoxyethylene alkyl ether sulfuric acid ester, a polyoxyethylenealkylaryl ether sulfate, alkyl sarcosinates, alpha olefin sulfonate sodium salt, alkyl benzene sulfonate or its salts, sodium lauroylsarcosinate, sulfosuccinates, polyacrylates, polyacrylates - free acid and sodium salt, salt of polyoxyethylenealkylaryl ether sulfuric acid ester, a polyoxyethylene alkyl ether phosphate, a salt of polyoxyethylenealkylaryl phosphoric acid ester, sulfosuccinates -mono and other diesters, phosphate esters, alkyl naphthalene sulfonate-isopropyl and butyl derivatives,alkyl ether sulfates - sodium and ammonium salts; alkyl aryl ether phosphates, ethylene oxides and its derivatives, a salt of polyoxyethylene aryl ether phosphoric acid ester, mono-alkyl sulphosuccinates, aromatic hydrocarbon sulphonates, 2-acrylamido-2-methylpropane sulfonic acid, ammonium laurylsulphate, ammonium perfluorononanoate, Docusate, Disodium cocoamphodiacetate, Magnesium laurethsulfate, Perfluorobutanesulfonic acid, Perfluorononanoic acid, carboxylates, Perfluorooctanesulfonic acid, Perfluorooctanoic acid, Phospholipid, Potassium lauryl sulfate, Soap, Soap substitute, Sodium alkyl sulfate, Sodium dodecyl sulfate, Sodium dodecylbenzenesulfonate, Sodium laurate, Sodium laurethsulfate, Sodium lauroylsarcosinate, Sodium myrethsulfate, Sodium nonanoyloxybenzenesulfonate, alkyl carboxylates, Sodium stearate, alpha olefin sulphonates, naphthalene sulfonate salts, alkyl naphthalene sulfonate fatty acid salts, naphthalene sulfonate condensates-sodium salt, fluoro carboxylate, fatty alcohol sulphates, alkyl naphthalene sulfonate condensates-sodium salt, a naphthalene sulfonic acid condensed with formaldehyde or a salt of alkylnaphthalene sulfonic acid condensed with formaldehyde; or salts, derivatives thereof.

Cationic surfactants include one or more of, but not limited to Dialkyl dimethyl ammonium chlorides, Alkyl methyl ethoxylated ammonium chlorides or salts, Dodecyl-, Coco-, Hexadecyl-, Octadecyl-, Octadecyl/Behenyl-, Behenyl-, Cocoamidopropyl-,Trimethyl Ammonium Chloride; Coco-, Stearyl-, bis(2-hydroxyethyl)Methyl Ammonium Chloride, Benzalkonium Chloride, Alkyl-, Tetradecyl-, Octadecyl-Dimethyl Benzyl Ammonium Chloride, Dioctyl-, Di(Octyl-Decyl)-, Didecyl-, Dihexadecyl-Distearyl-, Di(Hydrogenated Tallow)-Dimethyl Ammonium Chloride, Di(Hydrogenated Tallow) Benzyl-, Trioctyl-, Tri(Octyl-Decyl)-, Tridodecyl-, Trihexadecyl-Methyl Ammonium Chloride, Dodecyl Trimethyl-, Dodecyl Dimethyl Benzyl-, Di-(Octyl-Decyl) Dimethyl, Didecyl Dimethyl-Ammonium Bromide, quaternised amine ethoxylates, Behentrimonium chloride, Benzalkonium chloride, Benzethonium chloride, Benzododecinium bromide, Bronidox, quaternary ammonium salts Carbethopendecinium bromide, Cetalkonium chloride, Cetrimonium bromide, Cetrimonium chloride, Cetylpyridinium chloride, Didecyldimethylammonium chloride, Dimethyldioctadecyl ammonium bromide, Dimethyldioctadecyl-ammonium chloride, Domiphen bromide, Lauryl methyl gluceth-10 hydroxypropyldimonium chloride, Octenidinedihydrochloride, Olaflur, N-Oleyl-1, 3-propanediamine, Pahutoxin, Stearalkonium chloride, Tetramethylammonium hydroxide, Thonzonium bromide; salts or derivatives thereof.

The non-ionic surfactants include one or more of but not limited to polyol esters, polyol fatty acid esters, polyethoxylated esters, polyethoxylated alcohols, ethoxylated and propoxylated fatty alcohols, ethoxylated and propoxylated alcohols, EO/PO copolymers; EO and PO block copolymers, di, tri-block copolymers; block copolymers of polyethylene glycol and polypropylene glycol, poloxamers, polysorbates, alkyl polysaccharides such as alkyl polyglycosidesand blends thereof, amine ethoxylates, sorbitan fatty acid ester, glycol and glycerol esters, glucosidyl alkyl ethers, sodium tallowate, polyoxyethylene glycol, sorbitan alkyl esters, sorbitan derivatives, fatty acid esters of sorbitan (Spans) and their ethoxylated derivatives (Tweens), and sucrose esters of fatty acids, Cetostearyl alcohol, Cetyl alcohol, Cocamide DEA, Cocamide MEA, Decyl glucoside, Decylpolyglucose, Glycerol monostearate, Lauryl glucoside, Maltosides, Monolaurin, Narrow-range ethoxylate, Nonidet P-40, Nonoxynol-9, Nonoxynols, Octaethylene glycol monododecyl ether, N-Octyl beta-D-thioglucopyranoside, Octyl glucoside, Oleyl alcohol, PEG-10 sunflower glycerides, Pentaethylene glycol monododecyl ether, Polidocanol, Poloxamer, Poloxamer 407, Polyethoxylated tallow amine, Polyglycerol polyricinoleate, Polysorbate, Polysorbate 20, Polysorbate 80, Sorbitan, Sorbitanmonolaurate, Sorbitanmonostearate, Sorbitantristearate, Stearyl alcohol, Surfactin, glyceryl laureate, lauryl glucoside, nonylphenolpolyethoxyethanols, nonyl phenol polyglycol ether, castor oil ethoxylate, polyglycol ethers, polyadducts of ethylene oxide and propylene oxide, block copolymer of polyalkylene glycol ether and hydroxystearic acid, tributylphenoxypolyethoxy ethanol, octylphenoxypolyethoxy ethanol, etho-propoxylatedtristyrlphenols, ethoxylated alcohols, polyoxy ethylene sorbitan, fatty acid polyglyceride, a fatty acid alcohol polyglycol ether, acetylene glycol, acetylene alcohol, an oxyalkylene block polymer, polyoxyethylene alkyl ether, polyoxyethylenealkylaryl ether, a polyoxyethylenestyrylaryl ether, a polyoxyethylene glycol alkyl ether, polyethylene glycol, a polyoxyethylene fatty acid ester, a polyoxyethylenesorbitan fatty acid ester, a polyoxyethyleneglycerin fatty acid ester, Alcohol ethoxylates- C6 to C16/18 alcohols, linear and branched, Alcohol alkoxylates- various hydrophobes and EO/PO contents and ratios, Fatty acid esters-mono and diesters; lauric, stearic and oleic; Glycerol esters- with and without EO; lauric, stearic, cocoa and tall oil derived, Ethoxylated glycerine, Sorbitan esters- with and without EO; lauric, stearic and oleic based; mono and trimesters, Castor oil ethoxylates-5 to 200 moles EO; non-hydrogenated and hydrogenated, Block polymers, Amine oxides- ethoxylated and non-ethoxylated; alkyl dimethyl, Fatty amine ethoxylates- coco, tallow, stearyl, oleyl amines, a polyoxyethylene hydrogenated castor oil or a polyoxypropylene fatty acid ester; salts or derivatives thereof.

Amphoteric or Zwitterionic surfactants include one or more of, but not limited to one or more of betaine, coco and lauryl amidopropyl betaines, Coco Alkyl Dimethyl Amine Oxides, alkyl dimethyl betaines; C8 to C18, Alkyl dipropionates -sodium lauriminodipropionate, Cocoamidopropyl hydroxyl sulfobetaine, imidazolines, phospholipids phosphatidylserine, phosphatidylethanolamine, phosphatidylcholine, and sphingomyelins, Lauryl Dimethylamine Oxide, alkyl amphoacetates and proprionates, alkyl Ampho(di)acetates, and di-proprionates, lecithin and ethanolamine fatty amides; or salts, derivatives thereof.

Surfactants that are commercially available under the trademark but are not limited to one or more of Atlas G5000, TERMUL 5429, TERMUL 2510, ECOTERIC®, EULSOGEN® 118, Genapol®X, Genapol®OX -080, Genapol® C 100, Emulsogen ® EL 200, Arlacel P135, Hypermer 8261, Hypermer B239, Hypermer B261, Hypermer B246sf, Solutol HS 15, Promulgen™ D, Soprophor 7961P, Soprophor TSP/461, Soprophor TSP/724, Croduret 40, Etocas 200, Etocas 29, Rokacet R26, Cetomacrogol 1000, CHEMONIC OE-20, Triton N-101, Triton X-100, Tween 20, 40, 60, 65, 80, Span20, 40, 60, 80, 83, 85, 120, Brij®, Atlox 4912, Atlas G5000, TERMUL 3512, TERMUL 3015, TERMUL 5429, TERMUL 2510, ECOTERIC®, ECOTERIC® T85, ECOTERIC® T20, TERIC 12A4, EULSOGEN® 118, Genapol®X, Genapol®OX -080, Genapol® C 100, Emulsogen ® EL 200, Arlacel P135, Hypermer 8261, Hypermer B239, Hypermer B261, Hypermer B246sf, Solutol HS 15, Promulgen™ D, Soprophor 7961P, Soprophor TSP/461, Soprophor TSP/724, Croduret 40, Etocas 200, Etocas 29, Rokacet R26, CHEMONIC OE-20, Triton™ N-101, IGEPAL CA-630 and Isoceteth-20.

However, those skilled in the art will appreciate that it is possible to utilize other conventionally known surfactants without departing from the scope of the present invention. The surfactants are commercially manufactured and available through various companies.

The surfactant is present in an amount of 0.1% to 60% w/w of the total composition. According to an embodiment, the surfactant is present in an amount of 0.1% to 40% w/w of the total composition. According to an embodiment; the surfactant is present in an amount of 0.1% to 30% w/w of the total composition. According to a further embodiment, the surfactant is present in an amount of 0.1% to 20% w/w of the total composition. According to an embodiment, the surfactant is present in an amount of 0.1% to 10% w/w of the total composition.

According to an embodiment, the solvents used in the agricultural composition include water miscible solvents. The water miscible solvents include, but are not limited to 1, 4-Dioxane, Ethylene glycol, Glycerol, N-Methyl-2-pyrrolidone, 1,3-Propanediol, 1,5-Pentanediol, Propylene glycol, Triethylene glycol, 1,2-Butanediol, 1,3-Butanediol, 1,4-Butanediol, Dimethylformamide, Dimethoxyethane, Dimethyloctanamide, Dimethyldecanamide. However, those skilled in the art will appreciate that it is possible to utilize other water miscible solvents without departing from the scope of the present invention.

According to an embodiment, the solvent is present in an amount of 0.1% to 95% w/w of the total composition. According to an embodiment, the solvent is present in an amount of 0.1% to 60% w/w of the total composition. According to an embodiment, the solvent is present in an amount of 0.1% to 40% w/w of the total composition. According to an embodiment, the solvent is present in an amount of 0.1% to 30% w/w of the total composition.

According to an embodiment, the disintegrating agents which are used in the agricultural composition include, but not limited to one or more of inorganic water soluble salts e.g. sodium chloride, nitrate salts; water soluble organic compounds such as agar, hydroxypropyl starch, carboxymethyl starch ether, tragacanth, gelatin, casein, microcrystalline cellulose, cross-linked sodium carboxymethyl cellulose, carboxymethyl cellulose, carboxymethyl cellulose calcium, sodium tripolyphosphate, sodium hexametaphosphate, metal stearates, a cellulose powder, dextrin, methacrylate copolymer, Polyplasdone® XL-10 (crosslinked polyvinylpyrrolidone), poly(vinylpyrrolidone), , sulfonated styrene-isobutylene-maleic anhydride copolymer, salts of polyacrylates of methacrylates, starch-polyacrylonitrile graft copolymer, sodium or potassium bicarbonates/ carbonates or their mixtures or salts with acids such as citric and fumaric acid or salts, derivatives thereof. However, those skilled in the art will appreciate that it is possible to utilize different disintegrating agents without departing from the scope of the present invention. The disintegrating agents are commercially manufactured and available through various companies.

According to an embodiment, the disintegrating agent is present in an amount of 0.1% to 50% w/w of the composition. According to an embodiment, the disintegrating agent is present in an amount of 0.1% to 30% w/w of the composition. According to an embodiment, the disintegrating agent is present in an amount of 0.1% to 20% w/w of the composition. According to an embodiment, the disintegrating agent is present in an amount of 0.1% to 10% w/w of the composition.

According to an embodiment, the hydrophobic agents include one or more of but not limited to modified starch, hydrophobically modified silicates, bentonite, attapulgite, talc, metal stearates and fluorinated silanes. However, those skilled in the art will appreciate that it is possible to utilize different hydrophobic agents without departing from the scope of the present invention. According to an embodiment, the hydrophobic agent is present in the concentration of 0.1% to 50% w/w of the total composition.

According to an embodiment, the binding agents or binders which are used in the agricultural composition include, but not limited to one or more of proteins, lipoproteins, lipids, glycolipid, glycoprotein, carbohydrates such as monosaccharides, disaccharides, oligosaccharides and polysaccharides, complex organic substance, synthetic organic polymers or derivatives and combinations thereof. However, those skilled in the art will appreciate that it is possible to utilize different binding agents without departing from the scope of the present invention. The binding agents are commercially manufactured and available through various companies.

According to an embodiment, the binding agent is present in an amount of 0.1% to 50% w/w of the composition. According to further embodiment, the binding agent is present in an amount of 0.1% to 30% w/w of the composition. According to further embodiment, the binding agent is present in an amount of 0.1% to 20% w/w of the composition. According to further embodiment, the binding agent is present in an amount of 0.1% to 10% w/w of the composition.

According to an embodiment, the carriers which are used in the agricultural composition include, but are not limited to one or more of solid carriers or fillers or diluents. According to another embodiment, the carriers include mineral carriers, plant carriers, synthetic carriers, water-soluble carriers. However, those skilled in the art will appreciate that it is possible to utilize different carriers without departing from the scope of the present invention. The carriers are commercially manufactured and available through various companies.

The solid carriers include natural minerals like clay such as china clay, acid clay, kaolin such as kaolinite, dickite, nacrite, and halloysite, serpentines such as chrysotile, lizardite, antigorite, and amesite, synthetic and diatomaceous silicas, montmorillonite minerals such as sodium montmorillonite, smectites, such as saponite, hectorite, sauconite, and hyderite, micas, such as pyrophyllite, talc, agalmatolite, muscovite, phengite, sericite, and illite, silicas such as cristobaliteand quartz, such as attapulgite and sepiolite; vermiculite, laponite, pumice, bauxite, hydrated aluminas, perlite, sodium bicarbonate, volclay, vermiculites, limestone, natural and synthetic silicates, charcoal, silicas, wet process silicas, dry process silicas, calcined products of wet process silicas, surface-modified silicas, mica, zeolite, diatomaceous earth, derivatives thereof;. chalks (Omya ®), fuller's earth, loess, mirabilite, white carbon, slaked lime, synthetic silicic acid, starch, modified starch (Pineflow, available from Matsutani Chemical industry Co., Ltd.), cellulose, plant carriers such as cellulose, chaff, wheat flour, wood flour, starch, rice bran, wheat bran, and soyabean flour, tobacco powder, a vegetable powder polyethylene, polypropylene, poly(vinylidene chloride), methyl cellulose, hydroxypropyl cellulose, hydroxypropyl methylcellulose, sodium carboxymethyl cellulose, propylene glycol alginate, polyvinylpyrrolidone, carboxyvinyl polymer, casein sodium, sucrose, salt cake, potassium pyrophosphate, sodium tripolyphosphate, maleic acid, fumaric acid, and malic acid or derivatives or mixtures thereof. Commercially available Silicates are Aerosil brands, Sipemat brands as Sipernat ® 50S and CALFLO E, and kaolin 1777. However, those skilled in the art will appreciate that it is possible to utilize different solid carriers without departing from the scope of the present invention. The solid carriers are commercially manufactured and available through various companies.

According to an embodiment, the carrier is present in an amount of 0.1% to 98% w/w of the composition. According to further embodiment, the carrier is present in an amount of 0.1% to 80% w/w of the composition. According to further embodiment, the carrier is present in an amount of 0.1% to 60% w/w of the composition. According to further embodiment, the carrier is present in an amount of 0.1% to 40% w/w of the composition. According to further embodiment, the carrier is present in an amount of 0.1% to 20% w/w of the composition.

According to an embodiment, the anticaking agents which are used in the agricultural composition include, but are not limited to one or more of polysaccharides such as starch, mannose, galactose, poly(vinylpyrrolidone), fumed silica (white carbon), ester gum, a petroleum resin, Foammaster® Soap L sodium stearate, Brij® 700 polyoxyethylene (100) stearylether, Aerosol® OT-B sodium dioctyl sulfosuccinate, Silwet® L-77 silicone-polyether copolymer, sodium acetate, sodium metasilicate, sodium alkylsulfosuccinates, salts or derivatives thereof. However, those skilled in the art will appreciate that it is possible to utilize different anti caking agents without departing from the scope of the present invention. The anticaking agents are commercially manufactured and available through various companies. According to an embodiment, the anticaking agent is present in an amount of 0.01% to 20% w/w of the total composition.

According to an embodiment, the antifoaming agents or defoamers which are used in the agricultural composition include, but not limited to one or more of silica, siloxane, silicone dioxide, polydimethyl siloxane, alkyl polyacrylates, ethylene oxide/propylene oxide copolymers, polyethylene glycol, Silicone oils and magnesium stearate or derivatives thereof. Preferred antifoaming agents include silicone emulsions (such as, e.g., Silikon® SRE, Wacker or Rhodorsil® from Rhodia), long-chain alcohols, fatty acids, fluoro-organic compounds. However, those skilled in the art will appreciate that it is possible to utilize other conventionally known antifoaming agents without departing from the scope of the present invention. The antifoaming agents are commercially manufactured and available through various companies. According to an embodiment, the antifoaming agent is present in an amount of 0.01% to 20% w/w of the total composition.

According to an embodiment, the pH-adjusters or buffers or neutralizing agents which are used in the agricultural composition include both acids and bases of the organic or inorganic type and mixtures thereof. According to further embodiment, pH-adjusters or buffers or neutralizing agents include, but not limited to one or more of organic acids, inorganic acids and alkali metal compounds or salts, derivatives thereof. According to an embodiment, the organic acids include, but not limited to one or more of citric, malic, adipic, fumaric, maleic, succinic, and tartaric acid, or salts, derivatives thereof; and the mono-, di-, or tribasic salts of these acids or derivatives thereof. Alkali metal compounds include, but not limited to one or more of hydroxides of alkali metals such as sodium hydroxide and potassium hydroxide, carbonates of alkali metals such as sodium carbonate, hydrogencarbonates of alkali metals such as sodium hydrogencarbonate and alkali metal phosphates such as sodium phosphate and mixtures thereof. According to an embodiment, the salts of inorganic acids include, but not limited to one or more of alkali metal salts such as lithium chloride, sodium chloride, potassium chloride, lithium nitrate, sodium nitrate, potassium nitrate, lithium sulfate, sodium sulfate, potassium sulfate, sodium monohydrogen phosphate, potassium monohydrogen phosphate, sodium dihydrogen phosphate, potassium dihydrogen phosphate and the like. Mixtures can also be used to create a pH-adjusters or buffers or neutralizing agents. However, those skilled in the art will appreciate that it is possible to utilize other conventionally known pH-adjusters or buffers or neutralizing agents without departing from the scope of the present invention. The pH-adjusters or buffers or neutralizing agents are commercially manufactured and available through various companies.

According to an embodiment, the pH-adjusters or buffers are present in an amount of 0.01% to 20% w/w of the total composition. According to an embodiment, the pH-adjusters or buffers are present in an amount of 0.01% to 10% w/w of the total composition. According to an embodiment, the pH-adjusters or buffers are present in an amount of 0.01% to 5% w/w of the total composition. According to an embodiment, the pH-adjusters or buffers are present in an amount of 0.01% to 1% w/w of the total composition.

According to an embodiment, the spreading agents which are used in the agricultural composition include, but not limited to one or more of cellulose powder, dextrin, modified starch, crosslinked poly(vinylpyrrolidone), a copolymer of maleic acid with a styrene compound, a (meth)acrylic acid copolymer, a half ester of a polymer consisting of polyhydric alcohol with dicarboxylic anhydride, a water-soluble salt of polystyrenesulfonic acid, fatty acids, latex, aliphatic alcohols, vegetable oils such as cottonseed, or inorganic oils, petroleum distillates, modified trisiloxanes, polyglycol, polyethers, clatharatesor salts or derivatives thereof. However, those skilled in the art will appreciate that it is possible to utilize other conventionally known spreading agents without departing from the scope of the present invention. The spreading agents are commercially manufactured and available through various companies.

According to an embodiment, the spreading agent is present in an amount of 0.1% to 20% w/w of the total composition. According to an embodiment, the spreading agent is present in an amount of 0.1% to 5% w/w of the total composition.

According to an embodiment, the sticking agents which are used in the agricultural composition include, but not limited to one or more of paraffin, a polyamide resin, polyacrylate, polyoxyethylene, wax, polyvinyl alkyl ether, an alkylphenol-formalin condensate, fatty acids, latex, aliphatic alcohols, vegetable oils such as cottonseed, or inorganic oils, petroleum distillates, modified trisiloxanes, polyglycol, polyethers, clatharates, a synthetic resin emulsion or salts or derivatives thereof. However, those skilled in the art will appreciate that it is possible to utilize other conventionally known sticking agents without departing from the scope of the present invention. The sticking agents are commercially manufactured and available through various companies.

According to an embodiment, the sticking agent can be present in an amount of 0.1% to 30% w/w of the total composition. According to an embodiment, the sticking agent is present in an amount of 0.1% to 15% w/w of the total composition.

According to an embodiment, the stabilizers which are used in the agricultural composition include, but not limited to one or more of peroxide compounds such as hydrogen peroxide and organic peroxides, alkyl nitrites such as ethyl nitrite and alkyl glyoxylates such as ethyl glyoxylate, zeolite, antioxidants such as phenol compounds, phosphoric acid compounds and the like. However, those skilled in the art will appreciate that it is possible to utilize other conventionally known stabilizers without departing from the scope of the present invention. The stabilizers are commercially manufactured and available through various companies.

According to an embodiment, the stabilizer is present in an amount of 0.1% to 30% w/w of the total composition. According to an embodiment, the stabilizer is present in an amount of 0.1% to 20% w/w of the total composition. According to an embodiment, the stabilizer is present in an amount of 0.1% to 10% w/w of the total composition.

According to an embodiment, the preservatives which are used in the agricultural composition include but not limited to, one or more of bactericides, anti-fungal agents, biocides, anti-microbial agents, and antioxidant. Non limiting examples of preservatives include one or more of paraben, its esters and salts, propionic acid and its salts, 2,4-hexadienoic acid (sorbic acid) and its salt, formaldehyde and paraformaldehyde, 2-hydroxybiphenyl ether and its salts, inorganic sulfites and bisulfites, sodium iodate, chlorobutanol, dehydraacetic acid, formic acid, 1,6-bis(4-amidino-2-bromophenoxy)-n-hexane and its salts, 5-amino-1,3-bis(2-ethylhexyl)-5-methylhexahydropyrimidine, 5-bromo-5-nitro-1,3-dioxane, 2-bromo-2-nitropropane-1,3-diol, 2,4-dichlorobenzyl alcohol, N-(4-chlorophenyl)-N'-(3,4-dichlorophenyl) urea, 4-chloro-m-cresol, 2,4,4'-trichloro-2'-hydroxy diphenyl ether, 4-chloro-3,5-dimethyl phenol, 1,1'-methylene-bis(3-(1-hydroxy methyl-2,4-dioximidazolidin-5-yl)urea), poly(hexamethylenediguanide) hydrochloride, 2-phenoxyethanol, hexamethylenetetramine, 1-(3-chloroallyl)-3,5,7-triaza-1-azonia-adamantane chloride, 1(4-chlorophenoxy)-1-(1H-imidazol-1-yl)-3,3-dimethyl-2-butanone, 1,3-bis(hydroxymethyl)-5,5-dimethyl-2,4-imidazolidinedione, benzyl alcohol, octopirox, 1,2-dibromo-2,4-dicyanobutane, 2,2'-methylenebis(6-bromo-4-chlorophenol), bromochlorophene, dichlorophene, 2-benzyl-4-chlorophenol, 2-chloroacetamide, chlorhexidine, chlorhexidine acetate, chlorhexidine gluconate, chlorhexidine hydrochloride, 1-phenoxypropan-2-ol, N-alkyl(C12-C22)trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidinophenoxy)-n-hexane and its salts, glutaraldehyde, 5-ethyl-1-aza-3,7-dioxabicyclo(3.3.0)octane, 3-(4-chlorophenoxy)propane-1,2-diol, Hyamine, alkyl(C8-C18)dimethylbenzyl ammonium chloride, alkyl(C8-C18)dimethylbenzylammonium bromide, alkyl(C8-C18)dimethylbenzylammoniumsaccharinate, benzyl hemiformal, 3-iodo-2-propynyl butylcarbamate, sodium hydroxymethylaminoacetate, cetyltrimethylammonium bromide, cetylpyridinium chloride, and derivatives of 2H isothiazol-3-one (so-called isothiazolone derivatives) such as alkylisothiazolones (for example 2-methyl-2H-isothiazol-3-one, MIT; chloro-2-methyl-2H-isothiazol-3-one, CIT), benzoisothiazolones (for example 1,2-benzoisothiazol-3(2H)-one, BIT, commercially available as Proxel® types from ICI) or 2-methyl-4,5-trimethylene-2H-isothiazol-3-one (MTIT), C1-C4-alkyl para-hydroxybenzoate, an dichlorophene, Proxel® from ICI or Acticide® RS from Thor Chemie and Kathon® MK from Rohm & Haas, Bacto-100, thimerosal, Sodium Propinoate, Sodium Benzoate, Propyl Paraben,Propyl Paraben Sodium, Potassium Sorbate, Potassium Benzoate, Phenyl Mercuric Nitrate, Phenyl Etehyl Alcohol, Sodium, Ethylparaben, Methylparaben, Butylparaben, Bezyla Alcohol, Benzothonium Chloride, Cetylpyridinium Chloride, Benzalkonium Chloride, 1,2-benzothiazol-3-one, Preventol® (Lanxess®), Butylhydroxytoluene, potassium sorbate, iodine-containing organic compounds such as 3-bromo-2,3-diiodo-2-propenyl ethyl carbonate, 3-iodo-2-propynyl butyl carbamate, 2,3,3-triiodo allyl alcohol, and parachlorophenyl-3-iodopropargylformal; benzimidazole compounds and benzthiazole compounds such as 2-(4-thiazolyl)benzimidazole and 2-thiocyanomethylthiobenzo-thiazole; triazole compounds such as 1-(2-(2',4'-dichlorophenyl)-1,3-dioxolane-2-ylmethyl)-1H-1,2,4-triazole, 1-(2-(2',4'-dichloro phenyl)-4-propyl-1,3-dioxolane-2-ylmethyl)-1H-1,2,4-triazole, and $\alpha$-(2-(4-chlorophenyl) ethyl)-$\alpha$-(1,1-dimethyl ethyl)-1H-1,2,4-triazole-1-ethanol; and naturally occurring compounds such as 4-isopropyl tropolone (hinokitiol) and boraxor salts or derivatives thereof. Antioxidants includes but not limited to one or more of imidazole and imidazole derivatives (e.g. urocanic acid), 4,4'-thiobis-6-t-butyl-3-methylphenol, 2,6-di-t-butyl-p-cresol (BHT), and pentaerythrityltetrakis[3-(3,5,-di-t-butyl-4-hydroxyphenyl)]propionate; amine antioxidants such as N,N'-di-2-naphthyl-p-phenylenediamine; hydroquinoline antioxidants such as 2,5-di(t-amyl)hydroquinoline; phosphorus-containing antioxidants such as triphenyl phosphate, caro- tenoids, carotenes (e.g. $\alpha$-carotene, $\beta$-carotene, lycopene) and derivatives thereof, lipoic acid and derivatives thereof (e.g. dihydrolipoic acid), aurothioglucose, propylthiouracil and further thio compounds (e.g. thioglycerol, thiosorbitol, thioglycolic acid, thioredoxin, N-acetyl, methyl, ethyl, propyl, amyl, butyl, lauryl, palmitoyl, oleyl, $\gamma$-linoleyl, cholesteryl and glyceryl esters thereof), and salts thereof, dilaurylthiodipropionate, distearylthiodipropion- ate, thiodipropionic acid and derivatives thereof (esters, ethers, lipids, nucleotides, nucleosides and salts), and sulfoximine compounds (e.g. buthioninesulfoximi- nes, homocysteine sulfoximine, buthionine sulfones, penta-, hexa-, heptathioninesul- foximine) in very low tolerated doses (e.g. pmol/kg to pmol/kg), $\alpha$-hydroxy acids (e.g. citric acid, lactic acid, malic acid), humic acids, gallic esters (e.g. propyl, octyl and dodecyl gallate), unsaturated fatty acids and derivatives, hydroquinone and derivatives thereof (e.g. arbutin), ubiquinone and ubiquinol, and derivatives thereof, ascorbyl palmitate, stearate, di- palmitate, acetate, Mg ascorbyl phosphates, diso- diumascorbyl phosphate and sulfate, potassium ascorbyltocopheryl phosphate, isoascorbic acid and derivatives thereof, the coniferyl benzoate of benzoin resin, rutin, rutinic acid and derivatives thereof, disodium rutinyldisulfate, dibutylhydroxytoluene, 4,4-thiobis-6-tert-butyl-3-methylphenol, butylhydroxy anisole, p-octylphenol, mono-(di- or tri-) methyl benzylphenol, 2,6-tert-butyl-4-methylphenol, pentaerythritol-tetrakis 3-(3,5-di-tert-butyl-4-hydroxyphenyl)propionate, butylhydroxyanisol, nordihydroguaiaic acid, nordihydro-guaiaretic acid, trihydroxybutyrophenone, uric acid and derivatives thereof, mannose and derivatives thereof, selenium and selenium derivatives (e.g. selenomethionine), stilbenes and stilbene derivatives (e.g. stilbene oxide, trans-stilbene oxide). However, those skilled in the art will appreciate that it is possible to utilize other conventionally known preservatives without departing from the scope of the present invention. The preservatives are commercially manufactured and available through various companies.

According to an embodiment, the preservative or bactericides or anti-fungal agents or biocides or anti-microbial agents or antioxidant is present in an amount of 0.1% to 20% w/w of the total composition. According to further embodiment, the preservative or bactericides or anti-fungal agents or biocides or anti-microbial agents or antioxidant is present in an amount of 0.1% to 10% w/w of the total composition. According to further embodiment, the preservative or bactericides or anti-fungal agents or biocides or anti-microbial agents or antioxidant is present in an amount of 0.1% to 5% w/w of the total composition. According to further embodiment, the preservative or bactericides or anti-fungal agents or biocides or anti-microbial agents or antioxidant is present in an amount of 0.1% to 1% w/w of the total composition.

According to an embodiment, the structuring agents which are used in the agricultural composition include, but not limited to one or more of thickeners, viscosity modifiers, tackifiers, suspension aids, rheological modifiers or antisettling agents. A structuring agent prevents sedimentation of the active ingredient particles after prolonged storage.

According to an embodiment, the structuring agents which are used in the aqueous suspension composition include, but not limited to one or more polymers such as polyacrylics, polyacrylamides, polysaccharides, hydrophobically modified cellulose derivatives, co-polymers of cellulose derivatives, carboxyvinyl or polyvinyl pyrrolidones, polyethylenes, polyethylene oxide, polyvinyl alcohol and derivatives; clays such as bentonite clays, kaolin , smectite, attapulgites, attaclays with high surface area silica and natural gums such as guar gum, xanthan gum, gum Arabic, gum tragacanth, rhamsan gum, locust bean gum, carageenan, welan gum, veegum, gelatin, dextrin, collagen; polyacrylic acids and their sodium salts; the polyglycol ethers of fatty alcohols and polyethylene oxide or polypropylene oxide condensation products and mixtures thereof and include ethoxylated alkyl phenols (also designated in the art as alkylaryl polyether alcohols); ethoxylated aliphatic alcohols (or alkyl polyether alcohols); ethoxylated fatty acids (or polyoxyethylene fatty acid esters); ethoxylatedanhydrosorbitol esters (or polyethylene sorbitan fatty acid esters), long chain amine and cyclic amine oxides which are nonionic in basic solutions; long chain tertiary phosphine oxides; and long chain dialkyl sulfoxides, fumed silica, mixture of fumed silica and fumed aluminium oxide, swellable polymers, polyamides or its derivatives; polyols such as glycerine, poly(vinyl acetate), sodium polyacrylate, poly(ethylene glycol), phospholipid (for example, cephalin, and the like); stachyose, fructo-oligosaccharides, amylose, pectins, alginates, hydrocolloids and mixtures thereof. Also, celluloses such as hemicellulose, carboxymethylcellulose, ethylcellulose, hydroxyethylcellulose, hydroxy-methyl ethyl cellulose, hydroxyl ethyl propyl cellulose, methylhydroxyethylcellulose, methylcellulose; starches such, starch acetates, starch hydroxyethyl ethers, ionic starches, long-chain alkyl starches, dextrins, maltodextrin, corn starch, amine starches, phosphates starches, and dialdehyde starches; plant starches such as corn starch and potato starch; other carbohydrates such as pectin, dextrin, amylopectin, xylan, glycogen, agar, gluten, alginic acid, phycocolloids, or derivatives thereof. However, those skilled in the art will appreciate that it is possible to utilize other conventionally known structuring agents without departing from the scope of the present invention.

Preferred structuring agents include one or more of xanthan gum, aluminum silicate, methylcellulose, polysaccharide, alkaline earth metal silicate, gelatin, and polyvinyl alcohol. The structuring agents are commercially manufactured and available through various companies.

The structuring agent is present in an amount of 0.01% to 5% w/w of the composition. According to an embodiment, the structuring agent is present in an amount of 0.01% to 4% w/w of the composition. According to an embodiment, the structuring agent is present in an amount of 0.01% to 3% w/w of the composition. According to an embodiment, the structuring agent is present in an amount of 0.01% to 2% w/w of the composition. According to an embodiment, the structuring agent is present in an amount of 0.01% to 1% w/w of the composition. According to an embodiment, the structuring agent is present in an amount of 0.01% to 0.1% w/w of the composition.

According to an embodiment, the antifreezing agents or freezing point depressants used in the aqueous suspension composition include, but are not limited to one or more of polyhydric alcohols such as ethylene glycol, diethylene glycol, dipropylene glycol, propylene glycol, butyrolactone, N,N-dimethyl-formamide, glycerol, monohydric or polyhydric alcohols, glycol ethers, glycol ethers, glycol monoethers such as the methyl, ethyl, propyl and butyl ether of ethylene glycol, diethylene glycol, propylene glycol and dipropylene glycol, glycol diethers such as methyl and ethyl diethers of ethylene glycol, diethylene glycol and dipropyleneglycol.or urea, glycerol, isopropanol, propylene glycol monomethyl ether, di- or tripropylene glycol monomethyl ether or cyclohexanol , carbohydrates such as glucose, mannose, fructose, galactose, sucrose, lactose, maltose, xylose, arabinose, sorbitol, mannitol, trehalose, raffinose or derivatives thereof. However, those skilled in the art will appreciate that it is possible to utilize different antifreezing agents without departing from the scope of the present invention. The antifreezing agents are commercially manufactured and available through various companies. According to an embodiment, the antifreezing agent is present an amount of from 0.1% to 20%w/w of the total composition.

According to an embodiment, the penetrant which is used in the aqeuous suspension composition include, but not limited to one or more of alcohol, glycol, glycol ether, ester, amine, alkanolamine, amine oxide, quaternary ammonium compound, triglyceride, fatty acid ester, fatty acid ether, N-methyl pyrrolidone, dimethylformamide, dimethylacetamide, or dimethyl sulfoxide, polyoxyethylenetrimethylolpropanemonooleate,polyoxyethylenetrimethylolpropa nedioleate, polyoxyethylenetrimethylolpropanetrioleate, polyoxyethylenesorbitanmonooleate, polyoxyethylene sorbitol hexaoleate. However, those skilled in the art will appreciate that it is possible to utilize different penetrants without departing from the scope of the present invention.

The penetrants are commercially manufactured and available through various companies.

According to an embodiment, the ultraviolet absorbent is selected from, but not limited to one or more of 2-(2'-hydroxy-5'-methylphenyl)benzotriazole, 2-ethoxy-2'-ethyloxazalic acid bisanilide, succinic acid dimethyl-1-(2-hydroxyethyl)-4-hydroxy-2,2,6,6-tetramethylpiperidine polycondensate, benzotriazole compounds such as 2-(2'-hydroxy-5'-methylphenyl)benzotriazole and 2-(2'-hydroxy-4'-n-octoxyphenyl)benzotriazole; benzophenone compounds such as 2-hydroxy-4-methoxybenzophenone and 2-hydroxy-4-n-octoxybenzophenone; salicylic acid compounds such as phenyl salicylate and p-t-butylphenyl salicylate; 2-ethylhexyl 2-cyano-3,3-diphenyl acrylate, 2-ethoxy-2'-ethyl oxalic bisanilide, and dimethyl succinate-1-(2-hydroxyethyl)-4-hydroxy-2,2,6,6-tetramethylpiperidine polycondensateor derivatives or the like. However, those skilled in the art will appreciate that it is possible to utilize different ultraviolet absorbents, without departing from the scope of the present invention. Such ultraviolet absorbents are commercially manufactured and available through various companies.

According to an embodiment, the UV ray scattering agents include, but not limited to titanium dioxide or the like may be used. However, those skilled in the art will appreciate that it is possible to utilize different UV ray scattering agents or mixtures thereof without departing from the scope of the present invention. Such UV ray scattering agents are commercially manufactured and available through various companies.

According to an embodiment, the humectant is selected from, but not limited to one or more of polyoxyethylene/polyoxypropylene copolymers, particularly block copolymers, such as the Synperonic PE series of copolymers available from Uniqema or salts, derivatives thereof. Other humectants are propylene glycol, monoethylene glycol, hexylene glycol, butylene glycol, ethylene glycol, diethylene glycol, poly (ethylene glycol), poly (propylene glycol), glycerol and the like; polyhydric alcohol compounds such as propylene glycol ether, derivatives thereof. Also other humectants include aloe vera gel, alpha hydroxyl acids such as lactic acid, glyceryl triacetate, honey, lithium chloride, etc. The non-ionic surfactants mentioned above also act as humectants. However, those skilled in the art will appreciate that it is possible to utilize other conventionally known humectants without departing from the scope of the present invention. The humectants are commercially manufactured and available through various companies.

According to an embodiment, the humectant is present in the range of 0.1% to 90% w/w of the total composition. According to an embodiment, the humectant is present in the range of 0.1% to 70% w/w of the total composition. According to an embodiment, the humectant is present in the range of 0.1% to 60% w/w of the total composition. According to an embodiment, the humectant is present in the range of 0.1% to 50% w/w of the total composition. According to an embodiment, the humectant is present in the range of 0.1% to 30% w/w of the total composition. According to an embodiment, the humectant is present in the range of 0.1% to 10% w/w of the total composition.

The inventors have further determined that the composition of the present invention surprisingly has enhanced physical properties of dispersibility, suspensibility, wettability, less viscosity, pourability, provides ease of handling and also reduces the loss of material while handling the product at the time of packaging as well as during field application. Surprisingly, the inventors have also determined that the agricultural composition in the form of liquid suspension and water dispersible granules display superior dispersion and ultimately efficacy even when higher concentration composition is used in the fields.

Dispersibility of the water dispersible granular agricultural composition is a measure of percent dispersion. Dispersibility is calculated by the minimum percent dispersion. Dispersibility is defined as the ability of the granules to disperse upon addition to a liquid such as water or a solvent. To determine dispersibility of the granular composition as per the standard CIPAC test, MT 174, a known amount of the granular composition was added to a defined volume of water and mixed by stirring to form a suspension. After standing for a short period, the top nine-tenths are drawn off and the remaining tenth dried and determined gravimetrically. The method is virtually a shortened test of suspensibility and is appropriate for establishing the ease with which the granular composition dispersed uniformly in water.

According to an embodiment, the water dispersible granules have a dispersibility of at least 30%. According to an embodiment, the water dispersible granules have a dispersibility of at least 40%. According to an embodiment, the water dispersible granules have a dispersibility of at least 50%. According to an embodiment, the water dispersible granules have a dispersibility of at least 60%. According to an embodiment, the water dispersible granules have a dispersibility of at least 70%. According to an embodiment, the water dispersible granules have a dispersibility of at least 80%. According to an embodiment, the water dispersible granules have a dispersibility of at least 90%. According to an embodiment, the water dispersible granules have a dispersibility of at least 99%. According to an embodiment, the water dispersible granules have a dispersibility of 100%.

According to an embodiment, the agricultural composition exhibits good suspensibility. Suspensibility is defined as the amount of active ingredient suspended after a given time in a column of liquid, of stated height, expressed as a percentage of the amount of active ingredient in the original suspension. The water dispersible granules can be tested for suspensibility as per the CIPAC Handbook, "MT 184 Test for Suspensibility" whereby a suspension of known concentration of the granular composition in CIPAC Standard Water was prepared and placed in a prescribed measuring cylinder at a constant temperature, and allowed to remain undisturbed for a specified time. The top 9/10ths were drawn off and the remaining 1/10th was then assayed chemically, gravimetrically, or by solvent extraction, and the suspensibility was calculated.

The suspensibility of the liquid suspension is the amount of active ingredient suspended after a given time in a column of liquid, of stated height, expressed as a percentage of the amount of active ingredient in the original suspension. The suspensibility of liquid suspension concentrate is determined as per CIPAC MT-161 by preparing 250 ml of diluted suspension, allowing it to stand in a measuring cylinder under defined conditions, and removing the top nine-tenths. The remaining tenth is then assayed chemically, gravimetrically or by solvent extraction, and the suspensibility is calculated.

According to an embodiment, the pesticidal composition in the form of water dispersible granules or liquid suspension has a suspensibility of at least 30%. According to an embodiment, the agricultural composition in the form of water dispersible granules and liquid suspension has a suspensibility of at least 40%. According to an embodiment, the agricultural composition has a suspensibility of at least 50%. According to an embodiment, the agricultural composition has a suspensibility of at least 60%. According to an embodiment, the agricultural composition has a suspensibility of at least 70%. According to an embodiment, the agricultural composition has a suspensibility of at least 80%. According to an embodiment, the agricultural composition has a suspensibility of at least 90%. According to an embodiment, the agricultural composition has a suspensibility of at least 99%. According to an embodiment, the agricultural composition has a suspensibility of 100%.

According to an embodiment, the pesticidal composition in the form of water dispersible granules, extruded granules, liquid suspension demonstrates superior stability towards heat, light, temperature and caking. According to further embodiment, the stability exhibited by the pesticidal composition is more than 3 years. According to further embodiment, the stability exhibited by the agricultural composition is more than 2 years. According to further embodiment, the stability exhibited by the pesticidal composition is more than 1 year. According to further embodiment, the stability exhibited by the pesticidal composition is more than 10 months. According to further embodiment, the stability exhibited by the pesticidal composition is more than 8 months. According to further embodiment, the stability exhibited by the pesticidal composition is more than 6 months.

According to an embodiment, the agricultural composition in the form of water dispersible granules exhibit superior wettability. Wettability is the condition or the state of being wettable and can be defined as the degree to which a solid is wetted by a liquid, measured by the force of adhesion between the solid and liquid phases. The wettability of the granular composition is measured using the Standard CIPAC Test MT-53 which describes a procedure for the determination of the time of complete wetting of wettable formulations. A weighed amount of the granular composition is dropped on water in a beaker from a specified height and the time for complete wetting was determined. According to another embodiment, the water dispersible granular composition has wettability of less than 2 minutes. According to another embodiment, the water dispersible granular composition has wettability of less than 1 minute. According to another embodiment, the water dispersible granular composition has wettability of less than 30 seconds.

According to an embodiment, the agricultural composition in the form of liquid suspension and water dispersible granules passes the wet sieve retention test. The test is used to determine the amount of non-dispersible material in formulations that are applied as dispersions in water. The wet sieve retention value of the agricultural composition in the form of liquid suspension and water dispersible granules is measured by using the Standard CIPAC Test MT-185 which describes a procedure for the measuring the amount of material retained on the sieve. A sample of the formulation is dispersed in water and the suspension formed is transferred to a sieve and washed. The amount of the material retained on the sieve is determined by drying and weighing.

According to an embodiment, the agricultural composition has a wet sieve retention value on a 75 micron sieve of less than 10%. According to an embodiment, the agricultural composition has a wet sieve retention value on a 75 micron sieve of less than 7%. According to an embodiment, the agricultural composition has a wet sieve retention value on a 75 micron sieve of less than 5%. According to an embodiment, the agricultural composition has a wet sieve retention value on a 75 micron sieve of less than 2%.

According to an embodiment, the agricultural composition in the form of liquid suspension is non-viscous and is easily pourable. The viscosity of a fluid is a measure of its resistance to gradual deformation by shear stress or tensile stress.

According to an embodiment, viscosity of the liquid suspension is determined as per CIPAC MT-192. A sample is transferred to a standard measuring system. The measurement is carried out under different shear conditions and the apparent viscosities are determined. During the test, the temperature of the liquid is kept constant. According to an embodiment, the agricultural composition in the form of liquid suspension composition has a viscosity at 25° C. of about 0.01 Pa.s (10 cps) to about 2 Pa.s (2000 cps) which makes it pourable. According to an embodiment, the agricultural composition in the form of liquid suspension composition has a viscosity at 25° C. of about 0.01 Pa.s (10 cps) to about 1.2 Pa.s (1200 cps). According to an embodiment, the liquid suspension composition has viscosity at 25° C. of about 0.01 Pa.s (10 cps) to about 0.5 Pa.s (500 cps). According to an embodiment, the liquid suspension composition has a viscosity at 25° C. of about less than 0.5 Pa.s (500 cps). According to an embodiment, the liquid suspension composition has viscosity at 25° C. of about 0.01 Pa.s (10 cps) to about 0.4 Pa.s (400 cps). According to an embodiment, the liquid suspension composition has viscosity at 25° C. of about 0.01 Pa.s (10 cps) to about 0.3 Pa.s (300 cps). Too viscous composition tends to form a cake making it unpourable and thus is undesirable.

According to an embodiment, the agricultural composition in the form of water dispersible granules and liquid suspension demonstrates superior stability in terms of suspensibility, dispersibility under accelerated storage condition (ATS). According to an embodiment, the agricultural composition demonstrates suspensibility of more than 90% under ATS. According to an embodiment, the agricultural composition demonstrates suspensibility of more than 80% under ATS. According to an embodiment, the agricultural composition demonstrates suspensibility of more than 70% under ATS. According to an embodiment, the agricultural composition demonstrates suspensibility of more than 60% under ATS. According to an embodiment, the agricultural composition demonstrates suspensibility of more than 50% under ATS. According to an embodiment, the agricultural composition demonstrates suspensibility of more than 40% under ATS. According to an embodiment, the agricultural composition demonstrates suspensibility of more than 30% under ATS.

According to an embodiment, the agricultural composition demonstrates dispersibility of more than 90% under ATS. According to an embodiment, the agricultural composition demonstrates dispersibility of more than 80% under ATS. According to an embodiment, the agricultural composition demonstrates dispersibility of more than 70% under ATS. According to an embodiment, the agricultural composition demonstrates dispersibility of more than 60% under ATS. According to an embodiment, the agricultural composition demonstrates dispersibility of more than 50% under ATS. According to an embodiment, the agricultural composition demonstrates dispersibility of more than 40% under ATS. According to an embodiment, the agricultural composition demonstrates dispersibility of more than 30% under ATS.

According to an embodiment, the agricultural composition demonstrates viscosity of less than 2 Pa.s (2000cps) under ATS. According to an embodiment, the agricultural composition demonstrates viscosity of less than 1.5 Pa.s (1500cps) under ATS. According to an embodiment, the agricultural composition demonstrates viscosity of less than 1.2 Pa.s (1200cps) under ATS. According to an embodiment, the agricultural composition demonstrates viscosity of less than 1 Pa.s (1000cps) under ATS. According to an embodiment, the agricultural composition demonstrates viscosity of less than 0.8 Pa.s (800cps) under ATS. According to an embodiment, the agricultural composition demonstrates viscosity of less than 0.5 Pa.s (500cps) under ATS. According to an embodiment, the agricultural composition demonstrates viscosity of less than 0.3 Pa.s (300cps) under ATS.

The invention further relates to the process of preparation of the agricultural compositions according to claims 1 and 2.

According to an embodiment, the invention relates to a process for preparing the agricultural composition comprising one or more of amino acid, their polymers, salts, derivatives or mixtures thereof, elemental sulphur and at least one surfactant in the form of water dispersible granules. The composition can further include at least one micronutrient. The agricultural composition in the form of water dispersible granules is made by various techniques such as spray drying, fluidized bed granulation, extrusion, freeze drying etc.

According to an embodiment, the process of preparing a water dispersible granular composition involves milling a blend of one or more of amino acid, their polymers, salts, derivatives or mixtures thereof; with elemental sulphur, and at least one surfactant to obtain slurry or a wet mix. The composition further includes at least one fertilizer, at least on additional active ingredient selected from micronutrients, plant growth promoters, pesticidal actives or mixtures thereof. The wet mix obtained is then dried, for instance in a spray dryer, fluid bed dryer or any suitable granulating equipment, followed by sieving to remove the under sized and oversized granules to obtain microgranules of the desired size.

According to another embodiment, the agricultural composition in the form of water dispersible granules is also made by dry milling one or more of amino acids, their polymers, salts or mixtures with elemental sulphur and optionally at least micronutrient in presence of at least one surfactant an at least one agrochemical excipient in an air mill or a jet mill to obtain the desired particle size in the range of 0.1 to 20 microns, preferably 0.1 to 10 microns. Water is added to the dry powder and the mixture is blended to obtain a dough or paste, which is then extruded through an extruder to obtain the granules of desired size.

According to another embodiment, the invention relates to a process for preparing the agricultural composition in the form of liquid suspension. According to further embodiment, the invention relates to a process for preparing the liquid suspension composition comprising one or more of amino acids, their polymers, salts, derivatives or mixtures thereof; and elemental sulphur, at least one structuring agent with at least one agrochemically acceptable excipient. The composition further includes at least one fertilizer, at least on additional active ingredient selected from micronutrients, plant growth promoter, pesticidal actives or mixtures thereof.

According to an embodiment, the process of preparing the liquid suspension composition involves homogenization of one or more of excipients by feeding them into a vessel provided with stirring facilities. The elemental sulphur and amino acids, their polymers, salts or derivatives or mixtures thereof optionally includes micronutrients are further added to the homogenised blend and stirred continuously for about 5 to 10 minutes until the total mixture becomes homogeneous. Subsequently, the suspension obtained is passed through the wet mill to obtain a particle size in the range of 0.1 to 20 microns, preferably 0.1 to 10 microns. Then, requisite quantity of the structuring agent is added to the obtained suspension, under continuous homogenization.

Mention is done to the use of the agricultural composition as at least one of a nutrient composition, a crop strengthener composition, a soil conditioner composition, crop fortification, and a yield enhancer composition.

Mention is done to the method of application of an effective amount of the agricultural composition wherein the composition is applied to the seeds, seedlings, crops, a plant, plant propagation material, locus, parts thereof or to the surrounding soil.

The composition is applied through a variety of methods. Methods of applying to the soil includes any suitable method, which ensures that the composition penetrates the soil, for example, nursery tray application, in furrow application, drip irrigation, sprinkler irrigation, soil drenching, soil injection, or incorporation into the soil, and such other methods.

According to an embodiment, the invention further relates to a method of improving the crop health, improving the crop nutrition by facilitating the uptake of essential nutrients, protecting the crop, enhancing the crop yield, strengthening the plant or conditioning the soil, improving soil microbial properties, the method comprising treating at least one of seeds, seedling, crops, a plant, plant propagation material, locus, parts thereof or to the surrounding soil with effective amount of the agricultural composition.

The composition of the invention can also be useful to improve, enhance or increase crop characteristics such as crop yield, crop nutrients uptake , emergence, plant vigour, bigger leaf blade, stronger and more productive tillers, pigment content, protein content, photosynthetic activity, earlier flowering, early seed germination, early grain maturity, increased shoot growth, improved water stress tolerance, and increased plant stand at a reduced application of the composition comprising the fertilizer, amino acid and optionally the micronutrient. The composition of the present invention can also be useful to mitigate plant stresses like saline stress, drought stress, heat stress, cold stress, salt stress, micro nutrient stress and any other biotic and abiotic stresses of crop plant.

According to an embodiment, the phrase "increased yield" of an agricultural plant means that the yield of a product of the respective plant is increased by a significant amount over the yield of the same product of the plant produced under the same conditions, but without the application of the agricultural composition of the invention. According to an embodiment, the phrase "increased nutrient uptake" means improved ion transport, plant metabolism which in turn increases the nutritional value of the fruits or vegetables. The vegetables or fruits are rich in nutritional elements such as minerals, proteins, vitamins, carbohydrates etc. According to an embodiment, "improved plant vigor" means improvement of certain characteristics of the crop or the plant such as delayed senescence, root growth, longer panicles, improved plant stand, the plant weight and height, improved visual appearance, improved vitality of the plant, improved quality of the plant, improved quality of the fruits or vegetables (or other products produced by the plant), improved defence mechanism of the plant such as induced tolerance against fungi, bacteria, viruses and/or insects.

The rates of application or the dosage of the composition depends on the type of use, the type of crops, target yield, or the specific active ingredients in the composition but is such that the agrochemical active ingredient, in an effective amount to provide the desired action (such as nutrient uptake plant vigor, crop yield).

## A. PREPARATION EXAMPLES:

The following examples illustrate the basic methodology and versatility of the composition of the invention. Amino acid, their polymers, or derivatives or salts is exemplified in the preparatory examples and can be replaced by any other amino acid, their polymers, salts, complexes or derivatives thereof. It should be noted that this invention is not limited to these exemplifications.

A. Water dispersible granular composition of elemental sulphur and amino acid, their polymers, salts, derivatives or mixtures thereof.

Example 1: Water dispersible granular composition of 25% Glycine and 55% Elemental sulphur: Water dispersible granular composition was prepared by blending 55 parts of elemental sulphur, 25 parts of glycine, 5 parts of naphthalene sulphonate condensate, 9 parts of maltodextrin, 3 parts of kaolin and 3 parts of silica to obtain a blend. The blend obtained was mixed with water in suitable mixing equipment and milled to form a slurry or wet mix.

The wet milled slurry obtained was spray dried at an inlet temperature less than 175°C and outlet temperature less than 90°C to get a granular powder. The composition had the following particle size distribution: D10 less than 2 microns; D50 less than 4 microns and D90 less than 9.5 microns. The granule size of the composition was in the range of 0.1-1.5 mm. The composition had dispersibility of 85%, suspensibility of 90%, wet sieve retention value of 0.8%, wettability of less than 30 sec. The composition further demonstrated dispersibility of 80% and suspensibility of about 86 %under accelerated storage condition.

Example 2: Water dispersible granular composition of 60% Lycine and 20% Elemental sulphur: This composition was prepared similar to Example 1 using 60 parts of lysine,20parts of elemental sulphur, 4parts of naphthalene sulfonic acid, 8 parts of starch and 3 parts of silica and 5 parts of bentonite. The composition had the following particle size distribution: D10 less than 9 micron; D50 less than 11 microns and D90 less than 20 microns. The granule size of the composition was in the range of 0.1-2.5mm. The composition had dispersibility of 90%, suspensibility of 95%,wet sieve retention value of 1.5% and wettability of less than 100 sec. The composition further demonstrated suspensibility of about 80%, dispersibility of 85%under accelerated storage condition.

Example 3: Water dispersible granular composition of 10% Histidine and 72% Elemental sulphur: This composition was prepared similar to Example 1 using 10 parts of histidine, 72 parts of elemental Sulphur, 8 parts of naphthalene sulfonate condensate, 4 parts of starch and 2 parts of silica and 4 parts of metal stearate. The composition had the following particle size distribution: D10 less than 3 micron; D50 less than 5 microns and D90 less than 11 microns. The granule size of the composition was in the range of 0.1-4mm. The composition had a dispersibility of 65%, suspensibility of 70%, wet sieve retention value of 1.5%and wettability of less than 140 sec. The composition further demonstrated suspensibility of about 70% and dispersibility of about 60% under accelerated storage condition.

Example 4: Water dispersible granular composition of 5.4% Proline and 90% Elemental sulphur: This composition was prepared similar to Example 1 using 5.4 parts of proline, 90parts of elemental sulphur, 2.3 parts of sodium alkyl naphthalene sulfonate, and 2 parts of silica and 0.3 parts of metal stearate. The composition had the following particle size distribution: D10 less than 6 micron; D50 less than 10 microns and D90 less than 16 microns. The granule size of the composition was in the range of 0.1-2.5mm. The composition had dispersibility of 69%, suspensibility of 75%,wet sieve retention value of 1.5%and wettability of less than 80 sec. The composition further demonstrated suspensibility of about 70% and dispersibility of about 65% under accelerated storage condition.

Example 5:Water dispersible granular composition of 10% Soya protein hydrolysate and 82% Elemental sulphur: This composition was prepared similar to Example 1 using 10 parts of soya protein hydrolysate,82 parts of elemental sulphur, 5 of maltodextrin, 3parts of sodium alkyl naphthalene sulfonate. The composition had the following particle size distribution: D10 less than 4 micron; D50 less than 6.5 microns and D90 less than 14 microns. The granule size of the composition was in the range of 0.1-3 mm. The composition had dispersibility of 60%, suspensibility of 65%,wet sieve retention value of 1.5%and wettability of less than 50 sec. The composition further demonstrated suspensibility of about 55% and dispersibility of about 60%under accelerated storage condition.

Example 6: Water dispersible granular composition of 20% Polylysine and 40% Elemental sulphur with micronutrients: This composition was prepared similar to Example 1 using 20 parts of polylysine, 20 parts of elemental sulphur, 6 parts of iron, 0.5 parts each of copper and molybdenum, 2 parts of manganese, 1 part of boron, 6 parts of zinc, 15 of polycarboxylate, 9 parts of benzene sulfonic acid, 10parts of lignin sulfonate, 10 parts of bentonite. The composition had the following particle size distribution: D10 less than 4 micron; D50 less than 6.5 microns and D90 less than 14 microns. The granule size of the composition was in the range of 0.1-2.5mm. The composition had dispersibility of 90%, suspensibility of 85%, wet sieve retention value of 1.5%and wettability of less than 50 sec. The composition further demonstrated suspensibility of about 80% and dispersibility of about 85%under accelerated storage condition.

Example 7: Water dispersible granular composition of 10% Fish protein hydrolysate and 70% Elemental sulphur: This composition was prepared similar to Example 1 using 10 parts of fish protein hydrolysate, 70 parts of elemental sulphur, 9 of polycarboxylate, 6 parts of lignin sulfonate, 3 parts of hydrophobically modified silica and 2 parts of kaolin. The composition had following particle size distribution: D10 less than 2.5 micron; D50 less than 5.5 microns and D90 less than 10 microns. The granule size of the composition was in the range of 0.1-1.0 mm. The composition had dispersibility of 70%, suspensibility of 65%, wet sieve retention value of 1.5%and wettability of less than 60 sec. The composition further demonstrated suspensibility of about 65% and dispersibility of about 60%under accelerated storage condition.

Example 8: Water dispersible granular composition of 10% Soya protein hydrolysate and 62.5% Elemental sulphur and 6% humic acid: This composition was prepared similar to Example 1 using 10 parts of soya protein hydrolysate, 62.5 parts of elemental sulphur, 6 parts of humic acid, 8 of polycarboxylate, 4 parts of benzene sulfonate, 5.5 parts of hydrophobically modified silica and 4 parts of kaolin. The composition had following particle size distribution: D10 less than 1.5 micron; D50 less than 3.5 microns and D90 less than 6 microns. The granule size of the composition was in the range of 0.1-1.0 mm. The composition had dispersibility of 75%, suspensibility of 75%, wet sieve retention value of 1.1% and wettability of less than 45 sec. The composition further demonstrated suspensibility of about 70% and dispersibility of about 70% under accelerated storage condition.

B. Liquid suspension compositions of amino acid, their salts, polymers or derivatives or mixtures thereof and elemental sulphur:

Example 9:Liquid Suspension composition of 10% Soya protein hydrolysate and 50% Elemental sulphur.

Liquid suspension composition was prepared by mixing 10 parts of soya protein hydrolysate, 50 parts of elemental sulphur, 6 parts of naptahlene sulfonate condensate, 4 parts of sorbitan monolaurate, 3 parts of ethylene glycol,24 parts of water and homogenised by feeding them into a vessel provided with stirring facilities until the total mixture was homogeneous. Subsequently, the suspension obtained was passed through the wet mill to obtain a suspension with less than 20microns particle size. Then, 3 part of guar gum (3%) was added under continuous homogenization to obtain the suspension concentrate. The composition had particle size distribution of about D10 less than 2.0 microns; D50 less than 3.5 microns and D90 less than 8 microns. The sample had suspensibility of about 85%, viscosity of about 0.55 Pa.s (550cps). The composition had suspensibility of about 79% and viscosity of about 0.6 Pa.s (600cps) under accelerated storage condition.

Example 10: Liquid Suspension composition of 3% Tryptophan and 60% Elemental sulphur: This composition was prepared similar to Example 9 using 3 parts of tryptophan, 60 parts of elemental sulphur, 9 parts of hydrophobically modified starch, 4 parts of polyethylene glycol, 2 parts of 3% solution of xanthan gum and 22 parts of water. The composition had the particle size distribution of about D10 less than 4 microns; D50 less than 7.5 microns and D90 less than 14 microns. The sample had suspensibility of about 60%, viscosity of about 1.1 Pa.s (1100cps). The composition had suspensibility of about 53% and viscosity of about 1.2 Pa.s (1200cps) under accelerated storage condition.

Example 11: Liquid Suspension composition of 60% Alanine and 10% Elemental sulphur: This composition was prepared similar to Example 9 using 60 parts of alanine, 10 parts of elemental sulphur, 5 parts of naphthalene sulfonate, 2 parts of polyethylene glycol, 2 parts of glycerol monolaureate, 1 parts of 3% solution of gum Arabic and 20 parts of water. The composition had particle size distribution of about D10 less than 1.5 microns; D50 less than 4 microns and D90 less than 7 microns. The sample had suspensibility of about 95%, viscosity of about 0.45 Pa.s (450cps). The composition had suspensibility of about 90% and viscosity of about 0.5 Pa.s (500cps) under accelerated storage condition.

Example 12: Liquid Suspension composition of 5% Proline and 55% Elemental sulphur: This composition was prepared similar to Example 9 using 5 parts of proline, 55 parts of elemental sulphur, 10 parts of sodium alkyl naphthalene sulfonate, 2 parts of polyethylene glycol, 3 parts of glycerol monolaureate, 2 parts of 3% solution of xanthan gum and 23 parts of water. The composition had particle size distribution of about D10 less than microns 5; D50 less than 7.5 microns and D90 less than 10 microns. The sample had suspensibility of about 79%, viscosity of about 0.85 Pa.s (850 cps). The composition had suspensibility of about 72% and viscosity of about 0.95 Pa.s (950cps) under accelerated storage condition.

Example 13: Liquid Suspension composition of 15% Glycine and 30% Elemental sulphur along with micronutrients: This composition was prepared similar to Example 9 using 15 parts of glycine, 30 parts of elemental sulphur, 3 parts iron, 3 parts zinc, 0.5 part each of molybdenum and copper and 1 part each of boron and manganese, 8 parts of maltodextrin, 7 parts of lingo sulfonate, 3 parts of 3% solution of tragacanth gum and 28 parts of water. The composition had particle size distribution of about D10 less than microns 2.5; D50 less than 4 microns and D90 less than 9 microns. The sample had suspensibility of about 90%, viscosity of about 0.35 Pa.s (350cps). The composition had suspensibility of about 86% and viscosity of about 0.42 Pa.s (420cps) under accelerated storage condition.

Example 13: Liquid Suspension composition of 5% Polyglutamic acid and 20% Elemental sulphur along with 5% micronutrients: This composition was prepared similar to Example 9 using 5 parts of polyglutamic acid, 20 parts of elemental sulphur, 1 parts iron, 2 parts zinc, 0.5 part each of molybdenum and copper, 0.5 part each of boron and manganese, 20 parts of sodium salt of a phenol sulphonic acid condensate, 9 parts of lignin sulfonate, 6 parts of bentonite, 5 parts of monoethylene glycol and 30 parts of water. The composition had particle size distribution of about D10 less than 5 microns; D50 less than 12 microns and D90 less than 16 microns. The sample had suspensibility of about 55%, viscosity of about 1.2 Pa.s (1200cps). The composition had suspensibility of about 48% under and viscosity of about 1.25 Pa.s (1250cps) under accelerated storage condition.

## FIELD STUDY:

**Experiment 1:** To assess the synergistic effect of different combination of elemental sulphur and amino acid on tomato.

The field trials were carried out to see the effect of different formulations of elemental sulphur and amino acids on chlorophyll content and yield parameters in commercially cultivated tomato at Nashik. The trial was laid out during rabi season in Randomized Block Design (RBD) comprising ten treatments as described below including untreated control, replicated three times. For each treatment, plot size of 35 sq.m (7m x 5m) was maintained. The treatments as per details given below were applied by drip irrigation at 20 days after planting of the tomato crop. The tomato crop in trial field was raised following good agricultural practice. The seedling of tomato, variety -Heemsohna, was used for the study and planted in 120 cm row to row and 45 cm plant to plant spacing.

Details of experiment

| | |
|---|---|
| a) Trial Location | : Nashik (Maharashtra) |
| b) Crop | : Tomato (Heemsohna) |
| c) Experiment season | : Rabi 2018-19 (November to March) |
| d) Trial Design | : Randomized Block Design |
| e) Replications | : Three |
| f) Treatment | : Ten |
| g) Plot size | : 7m x 5m = 35 sq.m |
| h) Date of sowing | : 10.11.2018 |

(continued)

| | |
|---|---|
| i) Date of Application | : 30.11.2018 |
| j) Method of application | : Fertigation (drip) |

The observation on different parameters viz. total chlorophyll content in leaves, % fruit set, was recorded at 30 days after application and fruit yield was recorded at harvest and mean data is presented in Table 1 to enumerate the synergistic impact of the combination comprising of elemental sulphur and amino acid vis-à-vis standalone treatment with elemental sulphur and amino acid on tomato fruit yield.

Table 1: To assess the synergistic effect of combination of elemental sulphur and amino acid on tomato

| Treatment details | Dose of compound in g.a.i./m$^2$ (g.a.i./acre) | | Total chlorophyll content in leaves (mg g-1 fresh weight) | Fruit set (%) | Fruit yield g/m$^2$ (Qtl/acre) | % Increase in Yield | |
|---|---|---|---|---|---|---|---|
| | Sulphur | Amino Acid | | | | Actual | Expected |
| T1: Elemental sulphur-90% WDG | 0.6177 (2500) | - | 0.462 | 79.5 | 8601.7383 (348.1) | 9 | - |
| T2: Glycine 50 % WP | - | 0.0617 (250) | 0.433 | 74.1 | 8300.2698 (335.9) | 5.2 | - |
| T3: Proline 40 % WP | - | 0.0308 (125) | 0.431 | 73.3 | 8391.6988 (339.6) | 6.4 | - |
| T4: Tryptophan 45% WP | - | 0.0370 (150) | 0.328 | 75.2 | 8507.8383 (344.3) | 7.8 | - |
| T5: Soya protein hydrolysate 50% WP | - | 0.0988 (400) | 0.471 | 80.1 | 8703.0515 (352.2) | 10.3 | - |
| T6*: Elemental sulphur-62.5% + Glycine 6.25% WDG as per embodiment of present invention | 0.6177 (2500) | 0.0617 (250) | 0.489 | 86.3 | 8703.0515 (376.1) | 17.8 | 13.7 |
| T7*: Elemental sulphur-90% + Proline 4.5% WDG as per embodiment of present invention | 0.6177 (2500) | 0.0308 (125) | 0.476 | 84.1 | 9182.436 (371.6) | 16.4 | 14.8 |
| T8*: Elemental sulphur-60% + Tryptophan 3.6% WDG as per embodiment of present invention | 0.6177 (2500) | 0.0370 (150) | 0.481 | 83.8 | 9254.0965 (374.5) | 17.3 | 16.1 |
| T9*: Elemental sulfur-62.5% + soya protein hydrolysate 10% WDG as per embodiment of present invention | 0.6177 (2500) | 0.0988 (400) | 0.498 | 87.4 | 9419.6571 (381.2) | 19.4 | 18.4 |
| T10: Untreated | - | - | 0.423 | 71.9 | 7890.0748 (319.3) | 0 | - |

It is often understood that the term "synergy" is as defined by Colby S. R. in an article entitled "Calculation of the synergistic and antagonistic responses of herbicide combinations" published in Weeds, 1967, 15, p. 20-22. The action expected for a given combination of two active components can be calculated as follows:

$$E = X + Y \ - XY/100$$

Where,

E= Expected % effect by mixture of two products X and Y in a defined dose.

X= Observed % effect by product A

Y= Observed % effect by product B

The synergy factor (SF) is calculated by Abbott's formula (Eq.(2)(Abbott, 1925).

$$SF= Observed\ effect\ /Expected\ effect$$

Where, SF >1 for Synergistic reaction; SF<1 for antagonistic reaction; SF=1 for additive reaction.

When the percentage of yield effect observed (E) for the combination is greater than the expected percentage, synergistic effect of the combination can be inferred. When the percentage of yield effect observed for the combination is equal to the expected percentage, merely an additive effect may be inferred, and wherein the percentage of yield effect observed for the combination is lower than the expected percentage, an antagonistic effect of the combinations can be inferred.

It was observed that combination of elemental sulphur and amino acid in the form of water dispersible granules is synergistic in nature. From the data presented in Table 1, it can be observed that treatments T6-T9 (water dispersible granules of elemental sulphur and amino acid) are synergistic in nature as compared to treatments T1-T5 (standalone) and treatment T10 (untreated). This synergistic nature of the treatments T6-T9 can be observed from the fruit yield of tomato crops. For instance, treatment T9 (elemental sulphur 62.5% + soya protein hydrolysate 10% WDG) had yield increase of about 19.4% whereas treatment T1 (elemental sulphur 90% WDG) and treatment T5 (soya protein hydrolysate 50% WP) had yield increase of about 9% and 10.3% respectively. Similarly, treatment T7 (elemental sulphur 90% + proline 4.5% WDG) had yield increase of about 16.4% whereas treatment T1 (elemental sulphur 90% WDG) and treatment T3 (proline 40% WP) had yield increase of about 9% and 6.4% respectively. Also, it can be observed that expected yield for treatment T6, T7 and T8 was about 13.7%, 14.8% and 16.1% respectively whereas actual yield was about 17.8%, 16.4% and 17.3% respectively. Thus, the combination of elemental sulphur and amino acid in WDG form as per embodiment of the present invention is synergistic and provides higher crop yield as compared to individual components.

**Experiment No 2:** To assess the impact of different type of formulation of elemental sulphur and soya protein hydrolysate on physiological and yield attributing parameters in Soybean.

The field trials were carried out to study the effect of different formulation of elemental sulphur and soya protein hydrolysate combinations on plant physiological and yield attributing parameters in soybean.

The trial was laid out during kharif season in Randomized Block Design (RBD) with six treatments including untreated control, replicated four times. For each treatment, plot size of 40 sq.m(8m x 5m) was maintained. The treatment samples as per treatment details given below were applied as basal application at the time of sowing of Soybean crop. The Soybean crop in trial field was raised following good agricultural practice. The seed of Soybean nut, variety JS 95-60, was used for the study and planted in 30cm row to row and 10 cm plant to plant spacing.

Details of experiment

a) Trial Location          : Hatod, Indore (MP)
b) Crop                    : Soybean (var: JS 95-60)
c) Experiment season       : Kharif 2018
d) Trial Design            : Randomized Block Design
e) Replications            : Four
f) Treatment               : Six
g) Plot size               :8m x 5m = 40sq.m
h) R x P spacing           : 30cm x 10cm
h) Date of sowing          : 23.06.2018
i) Date of Application     : 23.06.2018
j) Method of application   : Basal
k) Date of Harvesting      : 29.9.2018

The Nitrogen (N) content in leaves, chlorophyll contents in leaves and harvest data including pod number per plant, test weight, grain yield, protein content and oil contents in grain were assessed 60 days after planting and mean data is presented in Table 2 to enumerate the impact of different formulation types of elemental sulphur and soya protein hydrolysate on physiological and yield attributing parameters.

Table 2: To Study effect of combination of elemental sulphur + soya protein hydrolysate in the form of WDG, SC on soybean crop.

(A)

| Treatments | Product dose in g/m² (g/acre) | Dose of compound in g.a.i/g/m² (g.a.i/acre) | | N content in leaves (g/kg Dry Mass ) | Chloro phyll (a+b) content in mg/g Fresh weight | Pod numb er /plant | Test weig ht (in gm) |
|---|---|---|---|---|---|---|---|
| | | Sulph ur | Soya protein hydroly sate | | | | |
| T1: Untreated | - | - | - | 31.5 | 1.76 | 37.6 | 18.5 |
| T2:Elemental sulphur-90% WDG | 0.6864 (2778) | 0.617 7 (2500 ) | - | 35.3 | 1.92 | 52.3 | 19.7 |
| T3: Soya protein hydrolysate 50% WP | 0.1976 (800) | - | 0.0988 (400) | 36.7 | 1.98 | 49.7 | 19.4 |
| T4: Elemental sulphur- 62.5% + Soya protein hydrolysate 10% WDG as per embodiment of the present invention | 0.9884 (4000) | 0.617 7 (2500 ) | 0.0988 (400) | 38.1 | 2.11 | 62.8 | 20.1 |
| T5: Elemental sulphur- 62.5% + Soya protein hydrolysate 10% WP | 0.9884 (4000) | 0.617 7 (2500 ) | 0.0988 (400) | 33.9 | 1.86 | 45.2 | 19.2 |

| T6: Elemental sulphur-50% + Soya protein hydrolysate 8% SC as per embodiment of the present invention | 1.2355 (5000) | 0.6177 (2500) | 0.0988 (400) | 38.4 | 2.14 | 59.8 | 19.1 |
|---|---|---|---|---|---|---|---|

(B):

| Treatments | Product dose in g/m² (g/acre) | Dose of compound in g.a.ig/m² (g.a.i/acre) | | Grain yield in kg/m² (kg/acre) | % grain yield increase over control | | % Protein in seeds | Oil content in seed (%) |
|---|---|---|---|---|---|---|---|---|
| | | Sulphur | Soya protein hydrolysate | | Actual | Expected | | |
| T1: Untreated | - | - | - | 0.2292 (927.6) | 0 | - | 39.2 | 18.9 |
| T2: Elemental sulphur-90% WDG | 0.6864 (2778) | 0.6177 (2500) | - | 0.2723 (1102.3) | 18.8 | - | 41.9 | 21.3 |
| T3: Soya protein hydrolysate 50% WP | 0.1976 (800) | - | 0.0988 (400) | 0.2640 (1068.4) | 15.2 | - | 41.2 | 19.8 |

| T4: Elemental sulphur-62.5% + Soya protein hydrolysate 10% WDG as per embodiment of the present invention | 0.9884 (4000) | 0.6177 (2500) | 0.0988 (400) | 0.3053 (1235.7) | 33.2 | 31.2 | 43.7 | 21.9 |
|---|---|---|---|---|---|---|---|---|
| T5: Elemental sulphur-62.5% + Soya protein hydrolysate 10% WP | 0.9884 (4000) | 0.6177 (2500) | 0.0988 (400) | 0.2689 (1088.6) | 17.4 | 31.2 | 41.1 | 19.8 |
| T6: Elemental sulphur-50% + Soya protein hydrolysate 8% SC as per embodiment of the present invention | 1.2355 (5000) | 0.6177 (2500) | 0.0988 (400) | 0.3037 (1229.1) | 32.5 | 31.2 | 43.5 | 21.7 |

WDG=Water dispersible granule, SC= Suspension concentrate,

WP=Wettable powder

The combination of elemental sulphur and amino acid in form of water dispersible granule (WDG) and suspension concentrates (SC) is synergistic in nature. This synergistic behaviour of elemental sulphur + amino acid in the form of WDG and SC as per embodiment of the present invention can be observed from the grain yield in soybean crop. The three treatments namely T4 (elemental sulphur-62.5% +soya protein hydrolysate-10% WDG), T5 (elemental sulphur-62.5% +soya protein hydrolysate-10% WP) and T6 (elemental sulphur-50% +soya protein hydrolysate-8% SC) were applied at same active dosage i.e 0.6177gm/m$^2$ (2500gm/acre) of sulphur and 0.0988gm/m$^2$ (400gm/acre) of soya protein hydrolysate. Treatments T4 and T6 exhibits highest grain yield of about 0.3053kg/m$^2$ (1235.7 kg/acre) and 0.3037kg/m$^2$ (1229.1 kg/acre) respectively when compared to treatment T5 with a grain yield of 0.2689kg/m$^2$ (1088.6 kg/acre) as well as in comparison to stand alone treatment i.e. T3 (soya protein hydrolysate 50% WP) with grain yield of 0.2640 kg/m$^2$ (1068.4 kg/acre) and T2 (elemental sulphur 90%WDG) with grain yield of 0.2723 kg/m$^2$ (1102.3 kg/acre). The expected yield for treatments T4, T5 and T6 as seen from Table 2 is 31.2% but treatment T4, T5, T6 demonstrated yield increase of about 33.2%, 17.4% and 32.5% respectively. It was also observed that the nitrogen content in leaves with treatments T4 and T6 was about 38.1 g/kg dry mass and 38.4 g/kg dry mass respectively whereas with treatment T5 it was about 33.9 g/kg of dry mass. On other hand, treatments T1, T2 and T3 had nitrogen content of about 31.5g/kg, 35.3 g/kg and 36.7 g/kg of dry mass respectively.

Thus, the combination of elemental sulphur and soya protein hydrolysate in WDG and SC form as per embodiment of the present invention is synergistic in nature and provides higher crop yield as compared to combination of elemental sulphur and soya protein hydrolysate in the form of wettable powder and the individual treatments.

**Experiment 3:** To assess the effect of elemental sulphur and amino acid on growth and yield of Paddy.

The field trials were carried out on commercial cultivated field of paddy growing area at Karnal in Haryana to study the effect of different mixtures of elemental sulphur and amino acid and nutrients mixtures on yield attributing parameters in Paddy. The trial was laid out during kharif season in Randomized Block Design (RBD) with nine treatments including untreated control, replicated thrice.

Treatments:

T1 Untreated

T2 ES-62.5+ SPH 10%-WDG at 0.9884g/m$^2$ (4000g/acre)

T3 ES-35+ Glycine-25% + MN mixture-10% WDG at 0.9884g/m$^2$ (4000g/acre)

T4 ES-40% + Polyglutamic acid 8% +MN mixture-10% WDG at 0.9884g/m$^2$ (4000g/acre)

T5 ES-20%+ Polyglutamic acid 5 %+ MN mixture-5% SC at 0.9884g/m$^2$ (4000g/acre)

T6 ES-62.5% + SPH-10% + MN mixtures-10% WDG at 0.9884g/m$^2$ (4000g/acre)

T7 ES-62.5% + FPH-10% WDG at 0.9884g/m$^2$ (4000g/acre)

(MN mixtures -Fe-3%+ Zn-4% +Mn-1% +Bo-1%+Cu-0.5%+ Mo-0.5% ,

ES= Elemental Sulphur; SPH= Soya Protein Hydrolysate; FPH= Fish Protein Hydrolysate)

For each treatment, plot size of 40 sq.m (8m x 5m) was maintained. The test treatment samples as per treatment details mentioned above were applied via broad cast application after 10 days of paddy transplanting. The paddy crop in trial field was raised following good agricultural practice. The paddy seedling variety-PR 121 was used for planting in trial field and was planted in 45 cm row to row and 30 cm plant to plant spacing.

Details of experiment

| | |
|---|---|
| a) Trial Location | : Karnal (Haryana) |
| b) Crop | : Paddy- Variety PR 121 |
| c) Experiment season | : Kharif 2018 |
| d) Trial Design | : Randomized Block Design |
| e) Replications | : Three |
| f) Treatment | : Seven |
| g) Plot size | : 8m x 5m = 40sq.m |
| h) R x P spacing | : 45 cm x 30 cm |
| h) Date of planting | : 18.06.2018 |
| i) Date of Application | : 28.06.2018 |
| j) Method of application | : Soil application as top dressing |
| k) Date of Harvesting | : 05.10.2018 |

The observation on different physiological and yield attributing parameters viz. numbers of tillers and panicle bearing tillers, panicle length, numbers of grain/panicle, 1000 grain weight and grain yield was assessed at harvest and mean data is presented in Table 3 to enumerate the impact of different treatments on yield and yield attributing parameters on paddy .

**Table 3:** To study effect of mixtures comprising elemental sulphur, amino acid, micronutrients on Paddy

| Treatment | Product dose in g/m² (g/acre) | Number of tillering /sq.m | Number of panicle bearing tillering /sq.m | Panicle length (in cm) | Number of Grain/ panicle | 1000 grain weight (in gm) | Grain yield q/m² (q/acre) |
|---|---|---|---|---|---|---|---|
| T1 | - | 243.3 | 225.4 | 24.3 | 154.5 | 23.3 | 0.005 2 (21.3) |
| T2 | 0.9884 (4000) | 274.5 | 265.7 | 28.3 | 169.9 | 24.9 | 0.006 0 (24.3) |
| T3 | 0.9884 (4000) | 281.3 | 274.3 | 27.1 | 182.9 | 25.1 | 0.006 1 (24.8) |
| T4 | 0.9884 (4000) | 280.9 | 274.4 | 27.4 | 175.6 | 24.7 | 0.059 (24.1) |
| T5 | 0.9884 (4000) | 271.8 | 265.2 | 28.4 | 184.3 | 25.1 | 0.006 1 (24.9) |
| T6 | 0.9884 (4000) | 296.7 | 284.2 | 28.3 | 194.5 | 25.2 | 0.006 3 (25.7) |
| T7 | 0.9884 (4000) | 289.4 | 281.7 | 27.5 | 191.4 | 25.4 | 0.006 2 (25.3) |

It was observed from Table 3, that combination comprising elemental sulphur, amino acid and micronutrients contribute to the development and growth of crop. From Table 3, it was found that treatments T2-T7 as per the embodiment of the present invention depicted grain yield in range of about 0.0060q/m² (24.3q/acre) to 0.0063 q/m² (25.7 q/acre) whereas with treatment T1 (untreated) the grain yield was 0.0052q/m² (21.3q/acre). For instance treatment T6, T2 had grain yield of 0.0063q/m² (25.7q/acre), and 0.0060 q/m² (24.3 q/acre) respectively whereas treatment T1 had grain yield of about 0.0052q/m² (21.3 q/acre). It was thus noted that mixtures help improve not only the qualitative parameters such as number of tillers, panicle length etc. but also quantitative characteristic of the crop such as yield as compared to T1 (untreated).

**Experiment 4:** To assess the effect of elemental sulphur + amino acid or mixtures on soil pH and its effect on nutrient uptake on tomato plant.

Field trials were carried out at commercial poly house cultivated tomato field at Nashik in Maharashtra to study the effect of different mixtures of elemental sulphur and amino acids along with micronutrient mixtures on soil pH and to study its impact on nutrient content in tomato leaves. The trial was laid out during January to May 2019 spring season in Randomized Block Design (RBD) with seven treatments including untreated control, replicated thrice. Twenty tomato plants were selected for each treatment and replicated. The compositions of the present invention were applied via drip irrigation at 20 days after planting. The tomato crop in trial field was raised following good agricultural practice. The tomato seedling of variety Avinash was used for planting in trial field and planted in 120 cm row to row and 45 cm plant to plant spacing.

### Details of experiment

a) Trial Location          : Nasik (MH)
b) Crop                    : Tomato-Variety 'Avinash'
c) Experiment season       : Spring season (Jan -to - May 2019)
d) Trial Design            : Randomized Block Design
e) Replications            : Three
f) Treatment               : Seven
g) Plot size               : 8m x 5m = 40sq.m
h) Date of planting        : 09.01.2019
i) Date of Application      : .30.01.2019
j) Method of application   : Soil application by drip system

Soil pH was measured from the soil taken out from 10 cm around the tomato plant and 5 cm depth before treatment and 30 days after treatment. Nutrient content in tomato leaves were measured by collecting 3rd trifoliate leaves from each treatment at 30 days after treatment and mean of three replications is presented in Table 4.

**Table 4:** To assess the effect of elemental sulphur + amino acid or mixtures on soil pH and nutrient uptake on tomato plant

| Treatment details | Dose in mg/ plant | Soil pH | | Protein content in leaves (mg/ 100- g DW) | Nutrient content in leaves (DM) | | | | | | | Tomato fruit yield (kg/ plant ) |
|---|---|---|---|---|---|---|---|---|---|---|---|---|
| | | Just before application | 30 days after application | | N (%) | P (%) | K (%) | S (% ) | Zn (ppm) | Fe (ppm) | Mn (ppm) | |
| Untreated | - | 8.12 | 8.08 | 4.67 | 2.0 1 | 0.56 | 2.34 | 0.5 9 | 12.8 | 112. 3 | 90.3 | 1.98 |
| Elemental sulphur 90% WDG | 400 | 8.07 | 7.65 | 5.43 | 2.2 4 | 0.54 | 2.41 | 1.0 2 | 13.6 | 105. 3 | 83.3 | 2.36 |
| Glycine 50% WP | 100 | 8.14 | 7.9 | 5.01 | 2.8 1 | 0.52 | 2.32 | 0.6 2 | 12.9 | 116. 9 | 94.9 | 2.23 |
| Soya protein hydrolysat e 50 % WP | 100 | 8.14 | 8.02 | 5.22 | 2.8 4 | 0.51 | 2.5 | 0.6 8 | 13.4 | 110. 1 | 88.1 | 2.35 |
| Micro nutrient mixtures 10% WP | 400 | 8.20 | 7.98 | 5.45 | 2.1 9 | 0.53 | 2.45 | 0.6 2 | 15.1 | 130. 1 | 108. 3 | 2.48 |
| Treatment 1 | 400 | 8.03 | 7.01 | 5.65 | 3.1 7 | 0.54 | 2.31 | 1.3 4 | 13.1 | 114. 7 | 92.7 | 2.59 |
| Treatment 2 | 400 | 8.12 | 6.92 | 5.89 | 3.2 6 | 0.55 | 2.45 | 1.2 5 | 12.5 | 117. 4 | 95.4 | 2.62 |
| Treatment 3 | 400 | 8.17 | 7.02 | 5.71 | 3.1 9 | 0.53 | 2.39 | 1.3 9 | 19.5 | 144. 2 | 120. 9 | 2.77 |
| Treatment 4 | 400 | 8.09 | 6.88 | 5.92 | 3.0 8 | 0.57 | 2.28 | 1.4 3 | 19.2 | 148. 1 | 126. 1 | 2.84 |
| CD at 5% | | NS | 0.32 | 0.21 | 0.1 8 | 0.32 | 0.22 | 0.3 5 | 3.4 | 13.6 | 10.3 | 0.23 |

Treatment 1=Elemental sulphur-60% + Glycine 10 % WDG
Treatment 2=Elemental sulphur-60% + Soya protein hydrolysate10 % WDG
Treatment 3=Elemental sulphur-60% + Glycine 10 % + Fe-3%+ Zn-4% +Mn-1% +Bo-1%+Cu-0.5%+ Mo-0.5%) WDG
Treatment 4= Elemental sulphur-60% + Soya protein hydrolysate 10 % + Fe-3%+ Zn-4% +Mn-1%+Bo-1%+Cu-0.5%+ Mo-0.5%) WDG
Micro nutrient mixtures 10% WP= (Fe-3%+ Zn-4% +Mn-1% +Bo-1%+Cu-0.5% + Mo-0.5%)

From Table 4, it was noted that combination of elemental sulphur and amino acid plays an important role not only as a fertilizer or a nutrient but also act as a modulator to alter the conditions of soil and help in uptake of nutrient and amino acids which contribute to better protein content in the leaves of tomato plant. The individual compounds did not show any significant plant growth promoting activity whereas the combination comprising of elemental sulphur and amino acids surprisingly resulted in better nutrient uptake and protein content in tomato. For instance, it was observed that treatment 2 (elemental sulphur-60% + soya protein hydrolysate 10% WDG) and treatment 4 (elemental sulphur-60% + soya protein hydrolysate 10%+ Fe-3%+ Zn-4% +Mn-1%+Bo-1%+Cu-0.5%+ Mo-0.5%) WDG) had a protein content of about 5.89% and 5.92% respectively whereas elemental sulphur WDG and soya protein hydrolysate 50% WP had a protein content of 5.43% and 5.22% respectively. The yield with treatment T2, T4 was 2.62 and 2.84 kg/plant respectively whereas with elemental sulphur WDG, soya protein hydrolysate 50% WP was about 2.36 kg/plant, 2.35 kg/plant respectively. On comparing micronutrient uptake with T3, T4 and micro nutrient mixtures 10% WP treatment it was noted that the T3 and T4 treatments had better nutrient uptake. This is due to the fact that T3 and T4 treatments in form of WDG help optimize the soil pH and improve the nutrient uptake providing a highly nutritious fruit to the user. For example, iron uptake with T3, T4 was about 144.2 ppm and 148.1ppm respectively whereas with elemental sulphur WDG, Glycine 50% WP, soya protein hydrolysate 50% WP and micro nutrient mixtures 10% WP iron uptake was about 105.3 ppm, 116.9 ppm, 110.1 ppm and 130.1 ppm respectively. Thus, it can be noted from Table 4, that composition comprising of elemental sulphur, amino acid and optionally including micronutrients in form of water dispersible granules reduces the soil pH creating an environment which assist in better uptake of nutrients, improves protein content and yield of plants as compared to the stand alone treatments (elemental sulphur, amino acids, and micronutrient). Thus, the combination meets nutrient efficacy of crop and provides sulphur, nutrients and amino acids to the plants as a single solution.

**Experiment 5:** To study effect of particle size of combinations comprising elemental sulphur + amino acid + micronutrient mixtures on protein synthesis by plant leaves over a period of time.

The pot trial experiment were carried out to study the effect of different formulation of elemental sulphur and amino acid along with micronutrient at different particle size on protein synthesis in soybean over a period of time.

The earthen pots were field with two kilogram sandy loam soil and kept in a manner to maintain 4 replications of each treatment. Two soybean seeds were planted in each pot and sufficient soil moisture was maintained for proper plant growth.

The treatment details are as described below:

T1- ES 62.5% + SPH 10% + MN mixtures 10% WDG (0.1 to 20 micron) at 100mg/pot
T2- ES 62.5% + SPH 10% + MN mixtures 10% WDG (0.1 to 50 micron) at 100mg/pot
T3- ES 62.5% + SPH 10% + MN mixtures 10% WDG (20 to 50 micron) at 100mg/pot
T4- ES 62.5% + SPH 10% + MN mixtures 10% WDG (50 to 100 micron) at 100mg/pot
MN mixtures -Fe-3%+ Zn-4% +Mn-1% +Bo-1%+Cu-0.5%+ Mo-0.5%
ES=Elemental Sulphur
SPH= Soya protein hydrolysate

The experimental pots were kept at a temperature of 28 ± 2°C and sufficient moisture was maintained during entire experiment. The above mentioned treatments were applied to the soil in each pot after 20 days of soybean seed germination. Soybean leaf from top 3 trifoliate was picked up for assessment for total protein on 7, 14, 21, 28 and 35 days after treatment and is presented in Figure 1.

From the data represented in Figure1, it can be observed that treatment T1 (water dispersible granular composition of elemental sulphur-62.5% + soya protein hydrolysate 10% + micronutrient mixtures 10% WDG having particle size distribution in the range of 0.1 to 20 microns) prepared according to an embodiment of the present invention demonstrates significant uptake of protein than that of treatments T2 (water dispersible granular composition of elemental sulphur-62.5% + soya protein hydrolysate 10% + micronutrient mixtures 10% WDG having particle size distribution in the range of 0.1 to 50 microns), T3 (water dispersible granular composition of elemental sulphur-62.5% + soya protein hydrolysate 10% + micronutrient mixtures 10% WDG having particle size distribution in the range of 20 to 50 microns)and T4 (water dispersible granular composition of elemental sulphur-62.5% + soya protein hydrolysate 10% + micronutrient mixtures 10% WDG having particle size distribution in the range of 50 to 100 microns). For instance, it was observed that 7Days after application, protein content with T1 was about 3.5 gm/100g fresh weight whereas with T2, T3 and T4 it was about 3.2gm/100g, 3.1gm/100g and 3 g/100g fresh weight respectively. This shows that water dispersible granular composition of elemental sulphur-62.5% + soya protein hydrolysate 10% + micronutrient mixtures-10% WDG having particle size in the range of 0.1 to 20 microns makes the nutrients available for instant uptake by plants. Similar trend in protein content was observed even after 21 days of application of the treatments T1-T4.

It can be noted that, T1, T2, T3 and T4 are in the form of WDG formulation with same concentration of actives and also applied at same dosages but T1 with specific particle size of 0.1-20 microns prepared according to an embodiment of the present invention demonstrated higher protein content as compared to treatmentsT2, T3, and T4 with different particle size. Thus, it was surprisingly noted that even amongst WDG formulations, superior efficacy was observed with WDG formulation having specific particle size of 0.1-20 microns in comparison to WDG formulations having different particle size.

Further, the inventors of the present invention also tested the combination of elemental sulphur, amino acid with plant growth promoters on certain crops like paddy, tomato crops. It was observed that addition of plant growth promoter such as humic acid, fulvic acid, triacontanol to the combination of the present invention may further enhance crop characteristics like panicle length, plant height, grain or fruit yield and add to nutritional value of the crop.

Thus, it has been observed that the compositions of the present invention, demonstrate enhanced, efficacious and superior behaviour in the fields. In fact, various properties associated with the compositions according to the invention, include but are not limited to improved stability, improved toxicological and/or ecotoxicological behaviour, improved crop characteristics including crop yields, crop qualities such as improved nutrient content, more developed root system, increase in crop height, bigger leaf blade, less dead basal leaves, stronger tillers, greener leaf color, less fertilizers needed, tillering increase, increased shoot growth, improved plant or crop vigor, earlier flowering, more productive tillers, less plant verse (lodging), improved chlorophyll content of the leaves, photosynthetic activity, protein content, stress management, early seed germination, early grain maturity, improved quality of the produce, improved fortification of the crop, conditioning the soil, disease resistance and other advantages familiar to a person skilled in the art. Also, the compositions of the present invention are also suitable for drip irrigation or sprinkler irrigation in addition to other methods of applications of the agricultural compositions, in which most of the commercial products and prior art products fail.

Through the composition of the present invention, the number of applications or the amount of nutrients, fertilizers or pesticides are minimized. The composition is highly safe to the user and to the environment.

**Claims**

1.    A water dispersible granular composition for soil application comprising:

elemental sulphur in the range of 20-99%w/w of the total composition;
at least one amino acid, their polymer, salts or derivatives or mixtures thereof present in the range of 0. 1-70% w/w of the total composition;
at least one surfactant present in the concentration of 0.1-60% w/w of the total composition;
and wherein the granules of the composition comprise of particles in the size range of from 0.1 to 20 microns.

2.    A liquid suspension composition for soil application comprising:

elemental sulphur in the range of 1-65%w/w of the total composition;
at least one amino acid, their polymers, salts, derivatives or mixtures thereof present in the range of 0.1-70% w/w of the total composition;
at least one structuring agent present in the concentration of 0.01-5% w/w of the total composition;
and wherein composition has particle size in the range of from 0.1-20 microns.

3.    The composition as claimed in claim 1 or 2, comprises one or more of amino acids selected from alanine, arginine, aspartic acid, asparagine, citrulline, leucine, lysine, isoleucine, cysteine, glutamic acid, glutamine, glycine, gaba-aminobutyric acid, histidine, methionine, ornithine, proline, phenylalanine, serine, selenocysteine, valine, taurine, tyrosine, theanine, threonine, tryptophan, peptides, plant and animal protein, polyglutamic acid, protein hydrolysate, soya protein hydrolysate, whey protein hydrolysate, fish protein hydrolysate.

4.    The composition as claimed in claim 1 or 2, further comprises at least one micronutrient, their salts, derivatives or mixtures thereof present in the concentration of 0.1%-70% by weight of the total composition wherein the micronutrient is selected from zinc, iron, copper, boron, manganese, magnesium, silicone, cobalt, selenium, molybdenum, lithium, calcium, their salts, derivatives or mixtures thereof.

5.    The composition as claimed in claim 4, wherein the micronutrient comprises at least one water soluble micronutrient and wherein the molar ratio of amino acids to the metal ions of water soluble micronutrient is 2:1 to 38:1.

6.    The water dispersible granular composition as claimed in claim 1, wherein the ratio of sulphur to amino acid is 990:1 to 3.5:1.

7.    The liquid composition as claimed in claim 2, wherein the ratio of sulphur to amino acid is 650:1 to 1:70.

8.    The composition as claimed in claim 1 or 2 further comprises at least one plant growth promoter or fertilizer or pesticidal active.

9.    The composition as claimed in claim 8, wherein the plant growth promoter is selected from humic acid, fulvic acid, triaconatol, ascorbic acids, lactic acid, oxalic acid, citric acid, N-acetyl thiazolidine-4 carboxylic acid, paclobutrazol, phytic acid, fumaric acid, gibberelic acid, auxins or mixtures thereof present in the concentration of 0.1-60% w/w of the total composition.

10. The composition as claimed in claim 1, wherein the one or more agrochemically acceptable excipients is selected from one or more of fillers or carriers or diluents, spreading agents, colorants, binders, buffers or pH adjusters or neutralizing agents, antifoaming agents or defoamers, anti-settling agents, penetrants, preservatives, hydrophobic agents, ultraviolet absorbents, UV ray scattering agents, stabilizers and mixtures thereof.

11. The composition as claimed in claim 2, wherein the composition further comprises one or more of agrochemically acceptable excipients selected from one or more of colorants, water miscible solvents, pH adjusters, antifoaming agents, humectants, preservatives, UV ray scattering agents, antifreezing agent, stabilizers, sticking agents and spreading agents.

12. The composition as claimed in claim 1, wherein the granule size of the composition is in the range of 0.1 - 5 mm.

13. A process of preparation of the water dispersible granular agricultural composition as claimed in claim 1, wherein the process comprises:

a. milling a blend of elemental sulphur, at least one amino acid, their salts, derivatives and at least one surfactant to obtain a slurry or wet mix;
b. drying the wet mix to obtain the water dispersible granular composition; wherein the granules of the composition comprise of particles in the size range of 0.1 micron to 20 microns.

14. A process of preparation of the liquid suspension agricultural composition as claimed in claim 2, wherein the process comprises: milling a blend of elemental sulphur, at least one amino acid, their salts or derivatives or mixtures thereof, at least one structuring agent to obtain a slurry or wet mix with a particle size range of 0.1 micron to 20 microns.

15. The composition as claimed in claims 1 or 2, wherein the composition is as at least one of a fertilizer composition, a nutrient composition, a crop strengtheners composition, a soil conditioner composition, a yield enhancer composition.

16. A method of improving plant health or yield, the said method comprising treating at least one of a plant, a plant propagation material, locus or parts thereof, a seed, seedling or surrounding soil with the composition as claimed in claims 1 or 2.

**Patentansprüche**

1. Wasserdispergierbare granulatförmige Zusammensetzung zur Bodenanwendung, umfassend:

elementaren Schwefel im Bereich von 20-99 Gew.-% der Gesamtzusammensetzung;
mindestens eine Aminosäure, ihr Polymer, Salze oder Derivate oder Mischungen davon, die im Bereich von 0,1-70% Gew.-% der Gesamtzusammensetzung vorhanden ist/sind;
mindestens ein Tensid, das im Bereich von 0,1-60 Gew.-% der Gesamtzusammensetzung vorhanden ist;
und wobei das Granulat der Zusammensetzung Partikel im Größenbereich von 0,1 bis 20 Mikron umfasst.

2. Flüssige Suspensionszusammensetzung zur Bodenanwendung, umfassend:

elementaren Schwefel im Bereich von 1-65 Gew.-% der Gesamtzusammensetzung;
mindestens eine Aminosäure, ihre Polymere, Salze, Derivate oder Mischungen davon, die im Bereich von 0,1-70% Gew.-% der Gesamtzusammensetzung vorhanden ist/sind;
mindestens ein Strukturierungsmittel, das in einer Konzentration von 0,01-5 Gew.-% der Gesamtzusammensetzung vorhanden ist;
und wobei die Zusammensetzung eine Partikelgröße im Bereich von 0.1-20 Mikron aufweist.

3. Zusammensetzung nach Anspruch 1 oder 2, umfassend eine oder mehrere Aminosäuren, ausgewählt aus Alanin, Arginin, Asparaginsäure, Asparagin, Citrullin, Leucin, Lysin, Isoleucin, Cystein, Glutaminsäure, Glutamin, Glycin, gamma-Aminobuttersäure, Histidin, Methionin, Ornithin, Prolin, Phenylalanin, Serin, Selenocystein, Valin, Taurin, Tyrosin, Theanin, Threonin, Tryptophan, Peptide, pflanzliches und tierisches Protein, Polyglutaminsäure, Proteinhydrolysat, Sojaproteinhydrolysat, Molkenproteinhydrolysat, Fischproteinhydrolysat.

4. Zusammensetzung nach Anspruch 1 oder 2, weiterhin umfassend mindestens einen Mikronährstoff, deren Salze, Derivate oder Mischungen davon, der/die in einer Konzentration von 0,1 bis 70 Gew.-% der Gesamtzusammensetzung vorhanden ist/sind, wobei der Mikronährstoff aus Zink, Eisen, Kupfer, Bor, Mangan, Magnesium, Silizium, Kobalt, Selen, Molybdän, Lithium, Calcium, deren Salzen, Derivaten oder Mischungen davon ausgewählt ist.

5. Zusammensetzung nach Anspruch 4, wobei der Mikronährstoff mindestens einen wasserlöslichen Mikronährstoff umfasst und wobei das Molverhältnis von Aminosäuren zu den Metallionen des wasserlöslichen Mikronährstoffs 2:1 bis 38:1 beträgt.

6. Wasserdispergierbare granulatförmige Zusammensetzung nach Anspruch 1, wobei das Verhältnis von Schwefel zu Aminosäure 990:1 zu 3,5:1 beträgt.

7. Flüssige Zusammensetzung nach Anspruch 2, wobei das Verhältnis von Schwefel zu Aminosäure 650:1 bis 1:70 beträgt.

8. Zusammensetzung nach Anspruch 1 oder 2, die weiterhin mindestens einen Pflanzenwachstumspromotor oder Dünger oder pestiziden Wirkstoff umfasst.

9. Zusammensetzung nach Anspruch 8, wobei der Pflanzenwachstumspromotor aus Huminsäure, Fulvinsäure, Triaconatol, Ascorbinsäuren, Milchsäure, Oxalsäure, Citronensäure, N-Acetylthiazolidin-4-carbonsäure, Paclobutrazol, Phytinsäure, Fumarsäure, Gibberelinsäure, Auxinen oder Mischungen davon, der/die in einer Konzentration von 0,1 - 60 Gew.-% der Gesamtzusammensetzung vorliegt/vorliegen, ausgewählt ist.

10. Zusammensetzung nach Anspruch 1, wobei der eine oder die mehreren agrochemisch unbedenklichen Exzipienten aus Füllstoffen oder Trägern oder Verdünnungsmitteln, Spreitmitteln, Färbemitteln, Bindemitteln, Puffern oder Mitteln zum Einstellen des pH-Wertes oder Neutralisationsmitteln, Antischaummitteln bzw. Entschäumern, Antiabsetzmitteln, penetrationsverbessernden Mitteln, Konservierungsstoffen, hydrophoben Mitteln, Ultraviolettabsorptionsmittel, UV-Strahlstreumittel, Stabilisatoren und Mischungen davon ausgewählt ist/sind.

11. Zusammensetzung nach Anspruch 2, wobei die Zusammensetzung weiterhin einen oder mehrere agrochemisch unbedenkliche Exzipienten umfasst, ausgewählt aus einem oder mehreren der folgenden: Färbemitteln, wassermischbare Lösungsmitteln, Mitteln zum Einstellen des pH-Wertes, Entschäumungsmitteln, Feuchthaltemitteln, Konservierungsmitteln, UV-Strahlstreumittel, Frostschutzmitteln, Stabilisatoren, Haftmitteln und Spreitmitteln.

12. Zusammensetzung nach Anspruch 1, wobei die Granulatgröße der Zusammensetzung im Bereich von 0,1 - 5 mm liegt.

13. Verfahren zur Herstellung der wasserdispergierbaren granulatförmigen landwirtschaftlichen Zusammensetzung nach Anspruch 1, wobei das Verfahren Folgendes umfasst:

    a. das Mahlen einer Mischung aus elementarem Schwefel, mindestens einer Aminosäure, ihren Salzen, Derivaten und mindestens einem Tensid unter Erhalt einer Aufschlämmung oder Nassmischung;
    b. das Trocknen der Nassmischung unter Erhalt der wasserdispergierbaren granulatförmigen Zusammensetzung; wobei das Granulat der Zusammensetzung Partikel im Größenbereich von 0,1 Mikron bis 20 Mikron umfasst.

14. Verfahren zur Herstellung der flüssigen landwirtschaftlichen Suspensionszusammensetzung nach Anspruch 2, wobei das Verfahren Folgendes umfasst: das Mahlen einer Mischung aus elementarem Schwefel, mindestens einer Aminosäure, ihren Salzen oder Derivaten oder Mischungen davon und mindestens eines Strukturierungsmittels unter Erhalt einer Aufschlämmung oder Nassmischung mit einem Teilchengrößenbereich von 0,1 Mikron bis 20 Mikron.

15. Zusammensetzung nach Anspruch 1 oder 2, wobei die Zusammensetzung eine Düngemittelzusammensetzung und/oder eine Nährstoffzusammensetzung und/oder eine Pflanzenstärkungszusammensetzung und/oder eine Bodenkonditioniererzusammensetzung und/oder eine Ertragsverstärkerzusammensetzung ist.

16. Verfahren zur Verbesserung der Pflanzengesundheit oder des Ertrags, wobei das Verfahren die Behandlung von einer Pflanze, einem Pflanzenvermehrungsmaterial, einem Standort oder Teilen davon, einem Samen, einem Sämling und/oder dem umgebenden Boden mit der Zusammensetzung nach Anspruch 1 oder 2 umfasst.

**Revendications**

1. Composition granulaire dispersible dans l'eau pour application au sol comprenant :

   du soufre élémentaire dans la plage de 20 à 99 % p/p de la composition totale ;
   au moins un acide aminé, leurs polymère, sels ou dérivés ou mélanges de ceux-ci présents dans la plage de 0,1 à 70 % p/p de la composition totale ;
   au moins un tensioactif présent dans la concentration de 0,1 % à 60 % p/p de la composition totale ;
   et dans laquelle les granules de la composition sont composés de particules dans la plage de tailles allant de 0,1 à 20 microns.

2. Composition en suspension liquide pour application au sol comprenant :

   du soufre élémentaire dans la plage de 1 à 65 % p/p de la composition totale ;
   au moins un acide aminé, leurs polymères, sels, dérivés ou mélanges de ceux-ci présents dans la plage de 0,1 à 70 % p/p de la composition totale ;
   au moins un agent structurant présent dans la concentration de 0,01 à 5 % p/p de la composition totale ;
   et dans laquelle la composition a une taille de particule dans la plage allant de 0,1 à 20 microns.

3. Composition selon la revendication 1 ou 2, comprenant un ou plusieurs acides aminés choisis parmi l'alanine, l'arginine, l'acide aspartique, l'asparagine, la citrulline, la leucine, la lysine, l'isoleucine, la cystéine, l'acide glutamique, la glutamine, la glycine, l'acide gaba-aminobutyrique, l'histidine, la méthionine, l'ornithine, la proline, la phénylalanine, la sérine, la sélénocystéine, la valine, la taurine, la tyrosine, la théanine, la thréonine, le tryptophane, les peptides, les protéines végétales et animales, l'acide polyglutamique, l'hydrolysat de protéines, l'hydrolysat de protéines de soja, l'hydrolysat de protéines de lactosérum, l'hydrolysat de protéines de poisson.

4. Composition selon la revendication 1 ou 2, comprenant en outre au moins un micronutriment, leurs sels, dérivés ou mélanges de ceux-ci présents dans la concentration de 0,1 % à 70 % en poids de la composition totale, dans laquelle le micronutriment est choisi parmi le zinc, le fer, le cuivre, le bore, le manganèse, le magnésium, le silicium, le cobalt, le sélénium, le molybdène, le lithium, le calcium, leurs sels, dérivés ou mélanges de ceux-ci.

5. Composition selon la revendication 4, dans laquelle le micronutriment comprend au moins un micronutriment soluble dans l'eau et dans laquelle le rapport molaire des acides aminés sur les ions métalliques de micronutriment soluble dans l'eau est de 2:1 à 38:1.

6. Composition granulaire dispersible dans l'eau selon la revendication 1, dans laquelle le rapport de soufre sur acide aminé est de 990:1 à 3,5:1.

7. Composition liquide selon la revendication 2, dans laquelle le rapport de soufre sur acide aminé est de 650:1 à 1:70.

8. Composition selon la revendication 1 ou 2, comprenant en outre au moins un promoteur de croissance végétale ou un engrais ou un pesticide actif.

9. Composition selon la revendication 8, dans laquelle le promoteur de croissance végétale est choisi parmi l'acide humique, l'acide fulvique, le triaconatol, les acides ascorbiques, l'acide lactique, l'acide oxalique, l'acide citrique, l'acide N-acétylthiazolidine-4 carboxylique, le paclobutrazol, l'acide phytique, l'acide fumarique, l'acide gibbérélique, les auxines ou des mélanges de ceux-ci présents dans la concentration de 0,1 à 60 % p/p de la composition totale.

10. Composition selon la revendication 1, dans laquelle l'excipient ou les excipients acceptable(s) sur le plan agrochimique est/sont choisi(s) parmi un ou plusieurs parmi des charges ou supports ou diluants, des agents d'étalement, des matières colorantes, des liants, des tampons ou des agents d'ajustement du pH ou des agents neutralisants, des agents antimousses ou des antimousses, des agents antisédimentation, des agents pénétrants, des conservateurs, des agents hydrophobes, des agents d'absorption d'ultraviolet, des agents de diffusion de rayonnement UV, des stabilisants et des mélanges de ceux-ci.

11. Composition selon la revendication 2, dans laquelle la composition comprend en outre un ou plusieurs excipient(s) acceptable(s) sur le plan agrochimique choisi(s) parmi un ou plusieurs parmi des matières colorantes, des solvants miscibles à l'eau, des agents d'ajustement du pH, des agents antimousses, des humectants, des conservateurs, des agents de diffusion de rayonnement UV, un agent antigel, des stabilisants, des agents collants et des agents d'étalement.

12. Composition selon la revendication 1, dans laquelle la taille des granules de la composition est dans la plage de 0,1 à 5 mm.

13. Procédé de préparation de la composition agricole granulaire dispersible dans l'eau selon la revendication 1, dans lequel le procédé comprend :

 a. le broyage d'un mélange de soufre élémentaire, d'au moins un acide aminé, de leurs sels, dérivés et d'au moins un tensioactif pour obtenir une suspension ou un mélange humide ;
 b. le séchage du mélange humide pour obtenir la composition granulaire dispersible dans l'eau ; dans lequel les granules de la composition sont composés de particules dans la plage de tailles de 0,1 micron à 20 microns.

14. Procédé de préparation de la composition agricole en suspension liquide selon la revendication 2, dans lequel le procédé comprend : le broyage d'un mélange de soufre élémentaire, d'au moins un acide aminé, de leurs sels ou dérivés ou de mélanges de ceux-ci, d'au moins un agent structurant pour obtenir une suspension ou un mélange humide comportant une plage de tailles de particules de 0,1 micron à 20 microns.

15. Composition selon la revendication 1 ou 2, dans laquelle la composition est au moins l'une parmi une composition d'engrais, une composition de nutriment, une composition renforçant les cultures, une composition de conditionnement du sol, une composition améliorant le rendement.

16. Procédé d'amélioration de la santé ou du rendement de végétaux, ledit procédé comprenant le traitement d'au moins l'un parmi un végétal, un matériau de propagation végétale, un site ou des parties de ceux-ci, une graine, un semis ou le sol environnant avec la composition selon les revendications 1 ou 2.

**FIGURE 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170283334 A
- WO 03053883 A
- EP 2359693 A

**Non-patent literature cited in the description**

- **COLBY S. R.** Calculation of the synergistic and antagonistic responses of herbicide combinations. *Weeds*, 1967, vol. 15, 20-22